# EUROPÄISCHE PATENTSCHRIFT

(11) **EP 2 364 785 B1**
(45) Veröffentlichungstag und Bekanntmachung des Hinweises auf die Patenterteilung: **01.05.2013**
(21) Anmeldenummer: 11166779.6
(22) Anmeldetag: 23.09.2009
(51) Int. Cl.: A61M 15/08, A61M 15/00, B05B 11/00

(54) **Austragvorrichtung mit Sperrmitteln**
Application device with blocking means
Dispositif de distribution avec moyens de blocage

(30) Priorität: 07.10.2008 US 287269
(43) Veröffentlichungstag der Anmeldung: 14.09.2011
(62) Teilanmeldung aus: 09012057.7
(73) Patentinhaber: Aptar Radolfzell GmbH, 78315 Radolfzell (DE)
(72) Erfinder: Fuchs, Karl-Heinz, 78315, Radolfzell (DE); Umbeer, Volker, 75328 Schömberg (DE); Körner, Joachim, 88690, Uhldingen-Mühlhofen (DE); Cater, Miro, Daytona Beach, FL 32124 (US)
(74) Vertreter: Patentanwälte Ruff, Wilhelm, Beier, Dauster & Partner

(56) Entgegenhaltungen:
- EP-A- 0 472 985
- EP-A2- 1 125 637
- DE-A1- 4 027 391
- DE-A1- 4 133 274
- DE-A1- 19 807 921
- US-A- 4 674 652
- US-A- 4 871 092
- US-A- 5 878 916

## Beschreibung

### Anwendungsgebiet und Stand der Technik

Die Erfindung betrifft eine Austragvorrichtung für Medien nach dem Oberbegriff von Anspruch 1.

Ähnliche Austragvorrichtungen sind aus dem Stand der Technik bekannt. Sie dienen dem Austrag insbesondere pharmazeutischer Medien wie beispielsweise flüssiger Arzneimittel. Gattungsgemäße Austragvorrichtungen habe ein Gehäuse, in dem ein Austragmittel, beispielsweise eine Kolbenpumpe, vorgesehen ist, das in Reaktion auf eine manuelle Bewegung des Betätigungsgliedes das Medium aus einem Mediumreservoir bis zu einer Austragöffnung der Austragvorrichtung fördert, so dass das Medium an die Umgebung abgegeben wird. Zum Zweck der Betätigung ist üblicherweise eine Fingerauflage vorgesehen, die bestimmungsgemäß von einem Benutzer manuell relativ zum dem Gehäuse niedergedrückt wird, um den Austragvorgang auszulösen.

Insbesondere bei Medien, die je nach Anwendung und Dosierung auch eine schädliche Wirkung haben können, ist es zweckmäßig, die Möglichkeit eines Austrags nicht allein in das Ermessen des Bedieners zu stellen. Solche Austragvorrichtungen weisen daher ein verlagerbares Sperrglied auf, welches einen Austragvorgang dadurch unterbinden kann, dass es eine Betätigung des Betätigungsgliedes fallweise mechanisch blockiert. Dieses Sperrglied kann in Abhängigkeit verschiedener Einflussgrößen in seine Freigabestellung verlagert werden, beispielsweise in Reaktion auf eine Authentifizierung des Bedieners mittels eines Zahlencodes oder eines Fingerabdrucks oder in Reaktion auf den Ablauf eines vorgegebenen Zeitintervalls zwischen zwei Austragvorgängen.

Eine solche Austragvorrichtung ist beispielsweise aus der EP 1125637 A2 bekannt. Bei dieser ist im Bereich des Unterbodens das Sperrglied vorgesehen, welches durch einen Elektromagneten zwischen der Freigabestellung und der Sperrstellung hin- und herschaltbar ist.

Aus der WO 2006/095184 A1 ist ebenfalls eine Austragvorrichtung bekannt. Bei dieser ist ein Sperrglied vorgesehen, welches radial unter eine Betätigungshandhabe verlagert werden kann, wobei die Verlagerung in diese Sperrstellung durch eine Federkraft erfolgt, während die Überführung in die Freigabestellung über einen Elektromotor realisiert ist.

Aus der US 5,878,916 A ist ein Spender bekannt, der über einen mit Rastleitern realisierten Mechanismus aufweist, der einen Rückhub eines Austragkopfes nach Betätigung oder Teilbetätigung erst gestattet, sobald die Betätigung vollendet wurde und der Austragkopf verdreht wurde. Eine Vielzahl von Austragvorgängen in schneller Folge wird hierdurch unterbunden.

### Aufgabe und Lösung

Aufgabe der Erfindung ist es, eine gattungsgemäße Austragvorrichtung in Hinblick auf eine sichere Verhinderung missbräuchlicher Überdosierungen weiterzubilden.

Erfindungsgemäß wird dies durch eine Austragvorrichtung nach Anspruch 1 erreicht.

Eine Rückführsperre einer erfindungsgemäßen Austragvorrichtung führt dazu, dass nach Beginn einer Betätigung und der daraus resultierenden Überschreitung der Sperrzwischenstellung eine unmittelbare Rücküberführung des Betätigungsgliedes in die Ausgangslage verhindert wird. Diese Rückführung ist erst dann möglich, wenn das Betätigungsglied zuvor bis in die Endlage überführt wurde. Als Endlage ist im Zusammenhang mit dieser Weiterbildung eine Position des Betätigungsgliedes zu verstehen, die nach Entlasten des Betätigungsgliedes zur Herstellung der genannten Sperrstellung führt. Im Falle der nachfolgend noch erläuterten Ausgestaltung der Austragvorrichtung mit zwei Kulissenspurabschnitten ist die Endstellung beispielsweise erreicht, sobald die Nocke in den zweiten Kulissenspurabschnitt eingerückt wurde.

Die erfindungsgemäße Gestaltung verhindert einen Missbrauch durch einen Benutzer, der versucht, eine unzulässig hohe Flüssigkeitsmenge auszutragen, indem er ausgehend von der Ausgangslage lediglich einen Teilhub ausführt, um dadurch zu verhindern, dass das Sperrglied aufgrund der Durchführung eines vollständigen Hubes in die Sperrstellung überführt wird. Die Rückführsperre erzwingt, dass nach einer begonnenen Hubbewegung des Betätigungsgliedes dieses zwischenzeitlich auch bis in die Hubendlage überführt wird, um anschließend einen neuen Hub beginnen zu können. Dadurch ist nach Beginn einer Hubbewegung die Ausgangslage nur gemeinsam mit dem Sperrzustand erreichbar.

Eine besondere Ausführungsform der Rückführsperre sieht vor, dass bis zur Erreichung der Endlage keinerlei Rückhubbewegung möglich ist. Dies kann beispielsweise durch eine Rastleiter erreicht werden, die bis zur Erreichung der Endlage stets die fortschreitende Hubbewegung sichert und dadurch auch Teilrückhübe verhindert. Eine einfachere Ausgestaltung sieht vor, dass die Rückführsperre lediglich in einer definierten Sperrzwischenstellung wirkt, so dass nach einer Hubbewegung, die über die Sperrzwischenstellung hinausgeht, ein Rückhub stets nur bis zur Sperrzwischenstellung möglich ist.

Erfindungsgemäß weist das Austragmittel eine Pumpe mit einer volumenveränderlichen Pumpkammer auf, wobei die Pumpe derart gestaltet ist, dass eine Befüllung der Pumpkammer im Rahmen eines Rückhubs erst ab einer definierten Befüllungszwischenstellung erfolgt, wobei diese Befüllungszwischenstellung derart angeordnet ist, dass sie beim Rückhub erst dann erreicht wird, wenn sich das Betätigungsglied in einer Lage zwischen der Sperrzwischenstellung und der Ausgangslage befindet. Dies wird dadurch erreicht, dass die Pumpe über ein Einlassventil verfügt, welches erst nach Erreichen der Befüllungszwischenstellung öffnet.

Bei einer solchen Gestaltung ist demnach eine Pumpe vorgesehen, die im Zuge eines Rückhubs nicht kontinuierlich Medium aus einem Mediumreservoir ansaugt, sondern beispielsweise zunächst einen Unterdruck in der Pumpkammer aufbaut, während ein Einlassventil in die Pumpkammer noch geschlossen bleibt. Erst bei Erreichen der Befüllungszwischenstellung öffnet das Einlassventil, so dass durch den aufgebauten Unterdruck schlagartig Medium aus dem Reservoir in die Pumpkammer einströmt. Durch die Anordnung, bei der die Befüllungszwischenstellung der Pumpe erst erreicht werden kann, wenn das Betätigungsglied zwischen der Sperrzwischenstellung und der Ausgangslage angeordnet ist, wird erreicht, dass nach Überschreiten der Sperrzwischenstellung durch das Betätigungsglied in Hubrichtung eine Neubefüllung der Pumpkammer erst möglich ist, wenn zuvor durch Vollendung der Hubbewegung das Betätigungsglied bis in seine Endlage verlagert wurde, da erst die dadurch ermöglichte anschließende Rückhubbewegung in Richtung der Ausgangslage eine Rücküberschreitung der Sperrzwischenstellung in Rückhubrichtung ermöglicht. Damit ist gewährleistet, dass die Neubefüllung der Pumpkammer zwingend mit der Erreichung des Sperrzustandes verbunden ist. Eine missbräuchliche Verwendung durch einen Benutzer, der Teilhubbewegungen zwischen der Sperrzwischenstellung und der Endlage des Betätigungsgliedes durchführt, führt daher nicht zu einem Medienaustrag.

Die Rückführsperre kann bei einer Weiterbildung Rastmittel aufweisen, wobei ein erster Bestandteil der Rastmittel in Betätigungsrichtung ortsfest zum Gehäuse angeordnet ist und wobei ein zweiter Bestandteil der Rastmittel, der zum Zusammenwirken mit dem ersten Bestandteil ausgebildet ist, in Betätigungsrichtung ortsfest zum Betätigungsglied angeordnet ist.

Diese Rastmittel sind derart ausgebildet, dass der zweite Bestandteil der Rastmittel, der ortsfest am Betätigungsglied vorgesehen ist, den ersten Bestandteil nur in Hubrichtung, nicht aber in Rückhubrichtung, überschreiten kann. Bei einer Hubbewegung wird daher der zweite Bestandteil der Rastmittel, beispielsweise ein in radiale Richtung auslenkbares Schnappglied, an dem ersten Bestandteil, beispielsweise einer Schwelle, vorbeigeführt und dabei zwischenzeitlich ausgelenkt. Sobald dieser erste Bestandteil der Rastmittel vom zweiten Bestandteil überschritten wurde, ist eine Rückführung in die Ausgangslage ohne zwischenzeitliches Erreichen der Endlage des Betätigungsgliedes nicht mehr möglich. Die Gestaltung mit Rastmitteln ist einfach in der Herstellung und insbesondere kostengünstig. Als zweiter Bestandteil der Rastmittel können Nocken vorgesehen sein, die mit oben beschriebenen Nocken zum Eingriff in die Kulissenspur identisch sein können oder versetzt zu diesen angeordnet sind. Der erste Bestandteil der Rastmittel, also beispielsweise eine Schwelle, kann im Bereich einer solchen Kulissenspur vorgesehen sein oder getrennt von der Kulissenspur angeordnet sein.

Um zu verhindern, dass die Wirkung der Rückführsperre dadurch umgangen wird, dass mit Gewalt das Betätigungsglied aus einer Teilhubstellung zurück in seine Ausgangsstellung gezogen wird, kann zwischen den von außen zugänglichen Flächen der Austragvorrichtung und dem zweiten Bestandteil der Rastmittel ein Ausgleichsglied vorgesehen sein, welches unter einer derartigen Gewalteinwirkung teleskopisch nachgibt und dadurch die Trennung der Rastmittel verhindert. Denkbar ist auch, dass das Ausgleichsglied als Sollbruchglied vorgesehen ist, welches unter Gewalteinwirkung zerbricht und dadurch dauerhaft weitere Betätigungsvorgänge verhindert.

Bei einer Weiterbildung der Erfindung ist ein erstes Federmittel vorgesehen ist, durch welches das Sperrglied in Richtung seiner Freigabestellung kraftbeaufschlagt wird und dass das Betätigungsglied und das Sperrglied derart miteinander wirkgekoppelt sind, dass in der Freigabestellung eine Hubbewegung des Betätigungsgliedes aus der unbetätigten Ausgangslage in die betätigte Endlage und/oder eine nachfolgende Rückhubbewegung aus der betätigten Endlage in die Ausgangslage mittels einer Übertragungsmechanik eine Energieeinspeisung in das erste Federmittel bewirkt.

Diese Weiterbildung führt dazu, dass sowohl die Energie zur Überführung des Sperrgliedes in die Sperrstellung als auch in die Freigabestellung aus der durch die Betätigung durch den Benutzer aufgebrachte Energie gespeist werden kann. So ist die Energie für die Freigabe im gesperrten Zustand im ersten Federmittel gespeichert. Sobald diese Energie freigegeben wird, bewegt sich das Sperrglied in seine Freigabestellung. Die Rücküberführung des Sperrgliedes wird zumindest abschnittsweise durch die Energie gespeist, die der Benutzer bei der Betätigung in das System einbringt. Vorzugsweise bewirkt die Bewegung des Betätigungsgliedes eine kontinuierliche Verlagerung des Sperrgliedes hin zur Sperrstellung oder darüber hinaus gegen die Kraft des ersten Federmittels und demnach bei gleichzeitigem Spannen des ersten Federmittels. Unter einer kontinuierlichen Verlagerung wird dabei eine Verlagerung verstanden, bei der eine fortschreitende Bewegung des Betätigungsgliedes in vorzugsweise proportionalem Maße gleichzeitig eine Verlagerung des Sperrgliedes bewirkt.

Diese Art der Wirkkopplung erlaubt es, dass Sperrglied durch die vom Bediener aufgebrachte Kraft in Richtung seiner Sperrstellung zu drücken, so dass ohne einen entsprechenden Motor und ohne eine Federkraftbeaufschlagung in Richtung der Sperrstellung alleine durch den Bediener die Sperrstellung wieder hergestellt wird. Die Bewegung des Betätigungsgliedes erfolgt vorzugsweise geradlinig, insbesondere vertikal bezogen auf eine bestimmungsgemäße Ausrichtung der Austragvorrichtung. Die Bewegungsrichtung des Sperrgliedes ist vorzugsweise etwa orthogonal zur Bewegungsrichtung des Betätigungsgliedes ausgerichtet, wobei die Wirkkopplung durch entsprechende Gleitschrägen oder Hebelanordnungen realisierbar ist.

Neben einer unmittelbaren kontinuierlichen Verlagerung des Sperrgliedes während der Betätigung kann die Einspeisung der Energie in das erste Federmittel auch mittelbar erfolgen, beispielsweise unter Nutzung eines weiteren Energiespeichers, der beispielsweise ebenfalls als Federmittel ausgebildet sein kann.

Besonders von Vorteil ist es, wenn sowohl die Hubbewegung als auch die Rückhubbewegung des Betätigungsgliedes mittels einer geeigneten Wirkkopplung genutzt werden, um die Verlagerung des Sperrgliedes zu verursachen. Dabei wird sowohl bei der Hubbewegung als auch bei der Rückhubbewegung eine Energieeinspeisung in das erste Federmittel erzielt. Durch diese Nutzung beider Bewegungen des Betätigungsgliedes relativ zum Gehäuse, ist eine vorteilhafte Übersetzung erzielbar, so dass das gleichzeitig mit der Bewegung des Betätigungsglieds stattfindende Spannen des Federmittels keine nennenswert erhöhte Betätigungskraft seitens des Bedieners erfordert.

Unter einem Federmittel im Sinne der Erfindung wird jedes Bauteil verstanden, welches mittels mechanisch gespeicherter Energie eine Kraftbeaufschlagung verursachen kann. Vorzugsweise handelt es sich um ein Bauteil, in dem die Energie durch elastische Verformung gespeichert ist, insbesondere um eine Schraubenfeder aus Metall oder Kunststoff.

Die durch die Bewegung des Betätigungsgliedes verursachte Bewegung des Sperrgliedes muss das Sperrglied nicht vollständig bis in die Sperrstellung bewegen. Es können auch weitere Mechanismen vorgesehen sein, die über einen Teil der Strecke die Kraftbeaufschlagung des Sperrgliedes in Richtung seiner Sperrstellung bewirken.

Das Betätigungsglied kann einstückig mit einem Abschnitt ausgebildet sein, der bestimmungsgemäß von einem Bediener im Zuge eines Austragvorgangs betätigt wird. Vorzugsweise ist das Betätigungsglied jedoch als separates Bauteil ausgebildet und mit der Betätigungshandhabe zum Zwecke der gemeinsamen Bewegbarkeit verbunden.

Das Austragmittel ist derart ausgebildet, dass durch Hinabdrücken des Betätigungsgliedes zumindest mittelbar ein Austragvorgang verursacht werden kann. Vorzugsweise handelt es sich beim Austragmittel um eine mechanisch mit der Betätigungshandhabe gekoppelte Verdrängerpumpe. Im Sperrzustand der Austragvorrichtung, bei der das Sperrglied in der Sperrstellung angeordnet ist, wird die Bewegungsfreiheit der Betätigungshandhabe oder des mit ihr verbundenen Betätigungsgliedes in einem ausreichenden Maße eingeschränkt, um eine Betätigung des Austragmittels vollständig oder annähernd vollständig zu verhindern.

Besonders bevorzugt ist es wenn das Betätigungsglied und das Sperrglied derart miteinander wirkgekoppelt sind, dass infolge der Bewegung des Betätigungsgliedes die Verlagerung des Sperrgliedes vorübergehend über die Sperrstellung hinaus erfolgt. Bei einer solchen Ausgestaltung ist die Wegstrecke, die das Sperrglied in Folge der Hubbewegung und/oder Rückhubbewegung des Betätigungsgliedes gegen die Federkraft des ersten Federmittels zurücklegt, größer als die Wegstrecke des Sperrgliedes zwischen der Sperrstellung und der Freigabestellung. Das Sperrglied wird demzufolge bei der Bewegung in seine Sperrstellung zunächst im Rahmen eines Überhubs über die Sperrstellung hinaus bewegt, um anschließend, beispielsweise bewirkt durch das erste Federmittel, zurück bis in die Sperrstellung gedrückt zu werden. Eine solche Gestaltung kann von Vorteil sein, um die jeweiligen Sperrabschnitte am Sperrglied und am Betätigungsglied, die in der Sperrstellung gemeinsam die Sperrwirkung entfalten, aneinander vorbeizuführen, so dass die Sperrstellung nach einem Austragvorgang zuverlässig wieder erreicht werden kann.

Die Wirkkopplung des Betätigungsgliedes mit dem Sperrglied wird vorzugsweise über eine Kulissenführung erreicht, mittels derer die Hubbewegung und/oder die Rückhubbewegung des Betätigungsgliedes die Verlagerung des Sperrgliedes bewirkt.

Unter einer solchen Kulissenführung wird jeder Mechanismus verstanden, bei dem ortsfest am Sperrglied und ortsfest am Betätigungsglied vorgesehene Abschnitte aneinander abgleiten, während das Betätigungsglied und das Sperrglied sich in unterschiedliche Richtungen zueinander bewegen. Die Bewegungsrichtung des Betätigungsgliedes und des Sperrgliedes schließen vorzugsweise einen Winkel von 90° ein. Vorzugsweise umfasst die Kulissenführung eine Kulissenspur am Sperrglied, die mit einer zum Eingriff vorgesehenen Nocke des Betätigungsgliedes zusammenwirkt. Aber auf die umgekehrte Konstellation ist realisierbar.

Besonders bevorzugt ist es dabei, wenn die Kulissenführung derartig ausgebildet ist, dass sie einen ersten Kulissenspurabschnitt aufweist, in den die Nocke bei der Hubbewegung einfährt, und/oder einen zweiten Kulissenspurabschnitt aufweist, in den die Nocke gegen Ende der Hubbewegung oder beim Übergang in die Rückhubbewegung einfährt.

Es ist dabei ausreichend, wenn einer der beiden genannten Führungsspurabschnitte vorgesehen ist, wobei in jedem Fall die Kulissenführung derartig ausgebildet sein muss, dass die Nocke nach Abschluss der Hubbewegung bei der nachfolgenden Rückhubbewegung einen anderen Bewegungspfad relativ zum Sperrglied beschreibt. Die Kulissenspurabschnitte können als Nut mit beidseitigen Kontaktflächen ausgebildet sein. Es reicht jedoch auch aus, wenn die Kulissenspurabschnitte lediglich einseitig eine Kontaktfläche aufweisen, gegen die die Nocke infolge der Kraft des Federmittels gedrückt wird. Bei einer Gestaltung mit zwei Kulissenspurabschnitten sind diese vorzugsweise in entgegengesetzte Richtung gegen die Betätigungsrichtung des Betätigungsgliedes geneigt, so dass die Hubbewegung und die Rückhubbewegung des Betätigungsgliedes eine gleichgerichtete Verlagerung des Sperrgliedes bewirken.

Um zu gewährleisten, dass die Rückhubbewegung der Nocke bezogen auf das Sperrglied auf einem Bewegungspfad erfolgt, der sich vom Bewegungspfad bei der Hubbewegung unterscheidet, sind vorzugsweise entsprechende Schaltmittel vorgesehen. Diese können dadurch realisiert sein, dass die Nocke im Zuge der Hubbewegung des Betätigungsgliedes quer zur Betätigungsrichtung elastisch ausgelenkt wird, wobei eine Stufe am Sperrglied vorgesehen ist, die im Zuge der Hubbewegung des Betätigungsgliedes bei gleichzeitiger Verminderung der elastischen Auslenkung der Nocke durch die Nocke überschritten wird. Die Stufe ist für die Nocke nur bei der Hubbewegung, nicht aber bei der gegenläufigen Rückhubbewegung überwindbar, so dass nach Überschreiten der Nocke im Zuge der Hubbewegung der Bewegungspfad der Nocke beim Rückhub bezogen auf das Sperrglied vom Bewegungspfad beim Hub unterscheidet. Vorzugsweise ist die Stufe in der Nähe des Umkehrpunktes vorgesehen, also im Bereich der letzten 40%, insbesondere der letzten 25% des Hubweges. Die elastische Auslenkung der Nocke erfolgt vorzugsweise in radialer Richtung und vorzugsweise mittels einer Rampenfläche, deren Fläche mit der Betätigungsrichtung des Betätigungsgliedes eine spitzen Winkel von weniger als 30 ° einschließt. Die elastische Auslenkung erfolgt vorzugsweise unmittelbar durch eine elastische Verformung der Nocke oder eines Stegs, an dem die Nocke vorgesehen ist. Alternativ können jedoch auch separate Federmittel wie beispielsweise eine Metallfeder vorgesehen sein.

Die Stufe stellt vorzugsweise den Übergangspunkt zwischen zwei Kulissenspurabschnitten dar, die in entgegengesetzte Richtung gegenüber der Betätigungsrichtung geneigt sind, um gemeinsam eine gleichgerichtete Bewegung des Sperrgliedes in Folge der Hub- und Rückhubbewegung des Betätigungsgliedes zu bewirken. Es sind jedoch auch Ausgestaltungen mit nur einem Kulissenspurabschnitt in oben beschriebener Art und Weise denkbar, bei denen die Hubbewegung des Betätigungsgliedes keinerlei Bewegung des Sperrgliedes bewirkt und erst der zweite Kulissenspurabschnitt nach Überwindung der Stufe oder einer anderweitigen Umschaltung durch die Schaltmittel gemeinsam mit der Nocke die Bewegung des Sperrgliedes im Zuge der Rückhubbewegung zur Folge hat. Alternativ kann es auch vorgesehen sein, das lediglich die Hubbewegung durch einen ersten Führungsspurabschnitt eine Verlagerung des Sperrgliedes zur Folge hat, während am Ende dieser ersten Führungsspur die Stufe vorgesehen ist, nach deren Überwindung durch die Nocke die Wirkkopplung zwischen Sperrglied und Betätigungsglied entfällt.

Im Sperrzustand wird eine Hubbewegung des Betätigungsgliedes vorzugsweise dadurch zumindest abschnittsweise unterbunden, dass mindestens ein sperrgliedseitiger Sperrabschnitt den Hubbewegungspfad von mindestens einem betätigungsgliedseitigen Sperrabschnitt versperrt, wobei es besonders bevorzugt ist, dass der betätigungsgliedseitige Sperrabschnitt identisch mit der Nocke der Kulissenführung ist. Bei einer solchen Gestaltung hat demnach die Nocke am Betätigungsglied eine Doppelfunktion. In der Sperrstellung des Sperrgliedes bildet sie den Sperrabschnitt, der eine Verlagerung des Betätigungsgliedes und somit einen Austrag unterbindet. In der Freigabestellung bildet die Nocke den betätigungsgliedseitigen Teil der Kulissenführung.

Die Erfindung wird ebenfalls durch eine gattungsgemäße Austragvorrichtung, insbesondere als Weiterbildung der vorbeschriebenen Austragvorrichtung verwirklicht, bei der eine Steueranordnung vorgesehen ist, die dafür ausgebildet ist, in einem Blockierzustand die Bewegung des Sperrgliedes gegenüber dem Gehäuse zu blockieren und durch eine mittels eines elektrischen Signals erzielbare Auslösung die Verlagerung des Sperrgliedes aus der Sperrstellung in die Freigabestellung zu ermöglichen.

Eine solche Steueranordnung erlaubt es demnach, das Sperrglied in der Sperrstellung zu halten, bis durch das elektrische Signal, welches beispielsweise der kurzzeitigen Bestromung eines Aktuators dienen kann, dieser Blockierzustand aufgehoben wird. Bei der oben beschriebenen Gestaltung mit einem ersten Federmittel, welches das Sperrglied zumindest mittelbar in Richtung der Freigabestellung kraftbeaufschlagt, führt die Auslösung zur Herstellung des Freigabezustandes.

Das elektrische Signal ist vorzugsweise nicht dafür ausgelegt, die tatsächlich zur Überführung des Sperrgliedes in die Freigabestellung erforderliche Energie zur Verfügung zu stellen, sondern ermöglicht stattdessen die Freigabe zuvor gespeicherter mechanischer Energie zu diesem Zweck.

Besonders bevorzugt ist es, wenn die Steueranordnung derart ausgebildet ist, dass sie bei einer Verlagerung des Sperrgliedes in die Sperrstellung automatisch in den Blockierzustand überführt wird.

Unter einer automatischen Überführung in den Blockierzustand wird in diesem Zusammenhang verstanden, dass ohne Eingreifen einer Elektronik, insbesondere durch einen ausschließlich mechanisch wirkenden Mechanismus die Verlagerung des Sperrgliedes in die Sperrstellung den Blockierzustand zur Folge hat. Dies kann beispielsweise erzielt werden, indem ein in der Sperrstellung wirkendes Rastmittel in Eingriff mit dem Sperrglied kommt. Ebenfalls möglich sind Gestaltungen, bei denen die Blockierung über Magnetkräfte oder Adhäsionskräfte zwischen dem Gehäuse und dem Sperrglied oder einem mit dem Sperrglied verbundenen Sperrhilfsglied genutzt werden.

Die Gestaltung hinsichtlich dieser Weiterbildung führt zu einer sehr einfachen Bauweise, da es nicht erforderlich ist, die Bewegung des Sperrgliedes in die Sperrstellung zu sensieren, um gezielt über die Elektronik der Austragsvorrichtung den Blockierzustand herzustellen.

Bei einer besonders bevorzugten Weiterbildung der Erfindung ist ein Riegelglied vorgesehen, welches im Blockierzustand der Steueranordnung eine Verlagerung des Sperrgliedes oder eines mit dem Sperrglied wirkverbundenen Sperrhilfsgliedes mechanisch blockiert. Im Zusammenhang mit dieser Erfindung wird unter einem Sperrhilfsglied ein Bauteil verstanden, welches derart mit dem Sperrglied verbunden ist, dass es sich bei einer Verlagerung des Sperrgliedes aus der Freigabestellung in die Sperrstellung in eine erste Richtung verlagert und bei einer Verlagerung des Sperrgliedes aus seiner Sperrstellung in die Freigabestellung in eine davon abweichende andere Richtung verlagert. Das Sperrhilfsglied kann derart zwangsgekoppelt sein, dass eine eindeutige Zuordnung zwischen jeder Stellung des Sperrgliedes und einer korrespondierenden Stellung des Sperrhilfsgliedes vorgesehen ist. Das Sperrglied und das Sperrhilfsglied können jedoch auch gedämpft, beispielsweise durch eine Federanordnung, miteinander verbunden werden, so dass trotz der grundsätzlichen Wirkkopplung eine begrenzte Beweglichkeit des Sperrhilfsgliedes auch bei sich nicht bewegendem Sperrglied besteht. Ein besonderer Vorteil bei der Verwendung eines Sperrhilfsgliedes liegt darin, dass die Beweglichkeit des Sperrhilfsgliedes anders geartet sein kann als die Beweglichkeit des Sperrgliedes. So kann insbesondere eine rotative Beweglichkeit des Sperrgliedes vorgesehen sein, wobei das Sperrglied mit einem Sperrhilfsglied wirkgekoppelt ist, welches seinerseits rein translativ beweglich ist.

Die oben beschriebene Weiterbildung mit einem Riegelglied gestattet eine besonders zuverlässige Sicherung des Blockierzustandes und eine einfache Herbeiführung der Auslösung. Als Riegelglied wird dabei ein Bauteil verstanden, welches mechanisch mit dem Sperrglied oder dem Sperrhilfsglied in Eingriff gebracht werden kann, um vorzugsweise formschlüssig die Bewegung des Sperrgliedes beziehungsweise Sperrhilfsgliedes zu unterbinden. Das Riegelglied kann dabei seinerseits selbst in Richtung der blockierenden Eingriffsstellung oder entgegen der Eingriffsstellung durch ein Federmittel kraftbeaufschlagt sein. Zur Herstellung des Blockierzustandes ist das Riegelglied vorzugsweise derart angeordnet und/oder ausgebildet, dass es durch die Überführung des Sperrgliedes in seine Sperrstellung in seinen Eingriffszustand gebracht wird. Dies kann beispielsweise dadurch erreicht werden, dass das Sperrglied oder das Sperrhilfsglied bei der Überführung in die Sperrstellung einen am Riegelglied vorgesehenen Fortsatz erfasst, der das Riegelglied in seiner Gesamtheit verlagert, insbesondere verschwenkt. Auch möglich ist eine Federkraftbeaufschlagung des Riegelgliedes in Richtung des Blockierzustandes. Das Riegelglied kann translativ beweglich sein. Als besonders vorteilhaft wird es jedoch angesehen, wenn das Riegelglied drehbar am Gehäuse gelagert ist.

Bei einer Weiterbildung der Erfindung ist im Blockierzustand der Steueranordnung das Sperrglied oder ein mit dem Sperrglied rückverbundenes Sperrhilfsglied durch einen Permanentmagneten in einer Lage gehalten, aus der resultierend das Sperrglied in seiner Sperrstellung angeordnet ist. Bei dieser beiden Weiterbildungen ist jeweils vorgesehen, dass ein Permanentmagnet zur Herstellung des Blockierzustandes der Steueranordnung vorgesehen ist. Bei einer Steueranordnung ohne Riegelglied ist entweder vorgesehen, dass das Sperrglied selbst oder ein Sperrhilfsglied bei Erreichen der Sperrstellung des Sperrgliedes durch einen Permanentmagneten derart kraftbeaufschlagt wird, dass eine Fixierung des Sperrgliedes bzw. des Sperrhilfsgliedes relativ zum Gehäuse erreicht wird.

Als Permanentmagnet wird in diesem Zusammenhang sowohl ein permanent magnetisiertes Bauteil verstanden als auch ein permanent stromdurchflossener Leiter, der dadurch ein Magnetfeld erzeugt. Vorzugsweise ist sowohl am entsprechenden beweglichen Glied, also am Sperrglied, dem Sperrhilfsglied, dem Riegelglied oder dem Riegelhilfsglied, als auch am Gehäuse jeweils ein Permanentmagnet vorgesehen. Es kann jedoch auch ausreichen, jeweils nur einen Permanentmagneten und statt des zweiten Permanentmagneten ein magnetisierbares Bauteil vorzusehen. Die im Kontakt dieser Erfindung besonders vorteilhafte Wirkung des Permanentmagneten liegt darin, dass die durch den Permanentmagneten hervorgerufene Kraft, die auf das Sperrglied, Sperrhilfsglied, Riegelglied oder Riegelhilfsglied wirkt, sehr schnell abnimmt, wenn das entsprechende Glied vom gehäusefesten Permanentmagneten entfernt wird, so dass die Wirkung des Permanentmagneten isoliert den Sperrzustand betrifft, während der Überführung in den Freigabezustand jedoch sehr bald vernachlässigbar klein wird.

Bei einer Gestaltung mit einem Permanentmagneten der mittelbar oder unmittelbar dafür verantwortlich ist, dass das Sperrglied in seiner Sperrstellung gehalten wird, ist es besonders bevorzugt, wenn die durch den oder die Permanentmagneten ausgeübte Kraft in einer Endlage, die der Sperrstellung des Sperrgliedes zugeordnet ist, größer ist, als die von Federmitteln der Austragvorrichtung in der Sperrstellung des Sperrgliedes in entgegengesetzte Richtung auf das Sperrglied, das Sperrhilfsglied, das Riegelglied bzw. das Riegelhilfsglied wirkende Kraft ist. Bei einer solchen Gestaltung ist dann, wenn sich das Sperrglied in der Sperrstellung befindet, eine Blockierung dadurch erreichbar, dass die Federkraft, die mittelbar oder unmittelbar das Sperrglied in Richtung der Freigabestellung oder das Riegelglied in Richtung eines entriegelten Zustandes drückt, geringer ist als die in dieser Stellung durch den Permanentmagneten bewirkte Kraft. Das entsprechende Glied wird dementsprechend durch den Permanentmagneten derartig stark kraftbeaufschlagt, dass die in entgegengesetzte Richtung wirkenden Federkräfte keine Bewegung des Sperrgliedes in die Freigabestellung oder des Riegelgliedes in seine entriegelte Stellung bewirken können. Erst wenn das entsprechende Glied durch eine zusätzliche Kraftbeaufschlagung ausreichend weit aus dem Wirkbereich des Permanentmagneten heraus bewegt wird, überwiegt die Federkraft und bewirkt somit unmittelbar oder mittelbar eine Überführung des Sperrgliedes in seine Freigabestellung.

Bei einer Weiterbildung der Erfindung ist zur Erzielung der Auslösung ein elektrisch ansteuerbarer Aktuator vorgesehen, vorzugsweise ein Elektromagnet, durch den entweder das Sperrglied oder ein mit dem Sperrglied wirkverbundenes Sperrhilfsglied kraftbeaufschlagt werden kann, so dass resultierend das Sperrglied in Richtung seiner Freigabestellung verlagert wird. Alternativ kann der elektrische ansteuerbare Aktuator zur Kraftbeaufschlagung des Riegelgliedes oder des mit dem Riegelglied wirkverbundenen Riegelhilfsgliedes Verwendung finden, wobei die Kraftbeaufschlagung in eine Richtung erfolgt, die zu einem Lösen der mechanischen Blockierung des Sperrgliedes durch das Riegelglied und damit zu einer Verlagerung des Sperrgliedes in Richtung seiner Freigabestellung führt. Der Aktuator gemäß dieser Weiterbildung ist dafür vorgesehen, in Reaktion auf eine Bestromung durch ein Steuergerät der Austragvorrichtung eine Kraftbeaufschlagung des entsprechenden Gliedes zu bewirken, um dieses in Richtung der Freigabestellung oder im Falle des Riegelgliedes in Richtung der gelösten Stellung zu verlagern. Dabei diente der Aktuator nicht der Einspeisung der vollständigen hierfür erforderlichen mechanischen Energie, sondern bewirkt lediglich eine Überwindung der zuvor herrschenden Blockierung, beispielsweise indem über einen kurzen Zeitraum, vorzugsweise weniger als 50 ms, eine Kraftbeaufschlagung entgegen der Kraftbeaufschlagung des zuvor beschriebenen Permanentmagneten erfolgt. Das entsprechende Glied, beispielsweise also das Sperrhilfsglied oder das Riegelhilfsglied, wird durch diese kurzzeitige Kraftbeaufschlagung von dem Permanentmagneten gelöst und/oder ausreichend weit aus dessen Wirkbereich entfernt, so dass nachfolgend eine Verlagerung des Riegelglieds in seine gelöste Stellung und/oder des Sperrgliedes in seine Freigabestellung durch die Kraftbeaufschlagung des ersten Federmittels und/oder eines anderen Federmittels erfolgen kann.

Die Verwendung eines Elektromagneten als Aktuator ist hierbei von besonderem Vorteil, da ein solcher Elektromagnet sehr preiswert und klein ausgebildet sein kann. Weiterhin ist die Ansteuerung eines solchen Elektromagneten durch eine Steuerelektronik der Austragvorrichtung denkbar einfach, da eine einfache kurzfristige Bestromung zur Erzielung des gewünschten Effekts ausreicht.

Zur Erreichung des oben beschriebenen Ziels ist es besonders vorteilhaft, wenn der elektrisch ansteuerbare Aktuator dafür ausgebildet ist, eine Kraft auf das Sperrglied, das Sperrhilfsglied, das Riegelglied oder das Riegelhilfsglied auszuüben, die größer ist als die in entgegengesetzte wirkende resultierende Kraft aus der auf das entsprechende Glied wirkenden Kraft des Permanentmagneten und vorzugsweise der Kraft eines das Glied kraftbeaufschlagenden Federmittels.

Durch eine solche Gestaltung, bei der das Steuergerät eine ausreichend starke Bestromung des Aktuator bestimmungsgemäß durchführt, kann eine entsprechende Bewegung des Gliedes entgegen der Kraft des Permanentmagneten erreicht werden, bis die resultierende auf das Glied wirkende Kraft in Richtung der gelösten Stellung bzw. der Freigabestellung größer ist als die in entgegengesetzter Richtung wirkende Kraft des Permanentmagneten.

Bei einer Weiterbilder der Erfindung ist vorgesehen, dass das Sperrglied zur Überführung aus der Sperrstellung in die Freigabestellung um eine in Betätigungsrichtung ausgerichtete Drehachse drehbar ausgebildet ist und über seinen Umfang verteilt mindestens drei Sperrabschnitte aufweist, die in der Sperrstellung des Sperrgliedes derart angeordnet sind, dass eine Hubbewegung des Betätigungsglieds verhindert wird.

Eine solche Ausgestaltung erlaubt eine besonders sichere Sperrstellung, da unabhängig davon, ob und in welche Richtung das Betätigungsglied quer zur Betätigungsrichtung der bestimmungsgemäßen Verwendung niedergedrückt wird, stets mindestens ein Sperrabschnitt vorhanden ist, der ein Bewegen des Betätigungsgliedes mit damit verbundenem Austragvorgang verhindert. Die Drehbeweglichkeit des Sperrgliedes ermöglicht es, diese besonders sichere Sperrstellung bei einer vergleichsweise einfachen mechanischen Gestaltung zu realisieren, da die Drehung eines einstückigen Sperrrings ausreicht, um gleichzeitig alle drei oder mehr Sperrabschnitte in die Sperrstellung zu verlagern. Als besonders bevorzugt wird es angesehen, wenn mindestens sechs Sperrabschnitte am Sperrglied vorgesehen sind und/oder wenn die Sperrabschnitte auf der Kreisbahn gleichmäßig verteilt sind.

Die Drehbeweglichkeit des Sperrgliedes um die Drehachse kann hinsichtlich des Drehwinkels begrenzt sein, so dass eine Überführung aus der Sperrstellung in die Freigabestellung und aus der Freigabestellung in die Sperrstellung in zueinander entgegengesetzten Richtungen durchgeführt wird. Alternativ ist es jedoch in der von Kugelschreibern bekannten Art und Weise auch möglich, die Drehbeweglichkeit des Sperrgliedes nicht zu begrenzen, sondern ein vollständiges Drehen des Sperrgliedes um seiner Drehachse zuzulassen, so dass die Überführung in die Sperrstellung und die Überführung in die Freigabestellung stets in die gleiche Drehrichtung erfolgt, wobei auf Seiten des Betätigungsgliedes und des Sperrgliedes mit jedem Freigabe- und Sperrzyklus andere Sperrabschnitte am Sperrglied und am Betätigungsglied in Kontakt miteinander gelangen, bis das Sperrglied und das Betätigungsglied relativ zueinander eine vollständige 360°-Drehung durchgeführt haben.

Besonders bevorzugt ist es, wenn das Sperrglied das Betätigungsglied umlaufend umgibt und die Drehachse des Sperrgliedes parallel zur Betätigungsrichtung und vorzugsweise koaxial zu einer Mittelachse der Austragvorrichtung ausgerichtet ist. Hierdurch sind besonders kompakte Bauweisen möglich. Das Sperrglied ist bei einer solchen Ausgestaltung in etwa ringförmig ausgebildet und weist eine mittige Ausnehmung auf, durch die hindurch das Betätigungsglied oder eine Betätigungshandhabe mit Austragmitteln der Austragvorrichtung verbunden ist.

Vorzugsweise weisen die sperrgliedseitigen Sperrabschnitte von einer Innenfläche des Sperrgliedes radial nach innen und die korrespondierenden betätigungsgliedseitigen Sperrabschnitte radial nach außen. Eine solche Ausgestaltung ist von Vorteil, da dabei eine Mantelfläche des Sperrgliedes, an deren Innenseite die Sperrabschnitte vorgesehen sind, einen Schutz gegen eine manuelle Beeinflussung durch einen Bediener darstellt. Die Sperrabschnitte, die in der Sperrstellung die Betätigung verhindern, sind durch die Mantelfläche schwer zugänglich und damit gut geschützt.

### Kurzbeschreibung der Zeichnungen

Weitere Vorteile und Merkmale der Erfindung ergeben sich außer aus den Ansprüchen auch aus der nachfolgenden Beschreibung von bevorzugten Ausführungsbeispielen der Erfindung, welche anhand der Darstellungen näher erläutert werden. Dabei zeigen:
- Figuren 1 bis 5: ein nicht von der Erfindung umfasster Spender, dessen Beschreibung als Grundlage für das Verständnis der erfindungsgemäßen Ausgestaltung dient, und
- Figuren 6a bis 6c: eine erfindungsgemäße Ausführungsform einer erfindungsgemäßen Austragvorrichtung in verschiedenen Stadien während der Betätigung und
- Fig. 7a und 7b: das Sperrglied der Ausführungsform gemäß der Fig. 2a bis 2c.

### Detaillierte Beschreibung der Ausführungsbeispiele

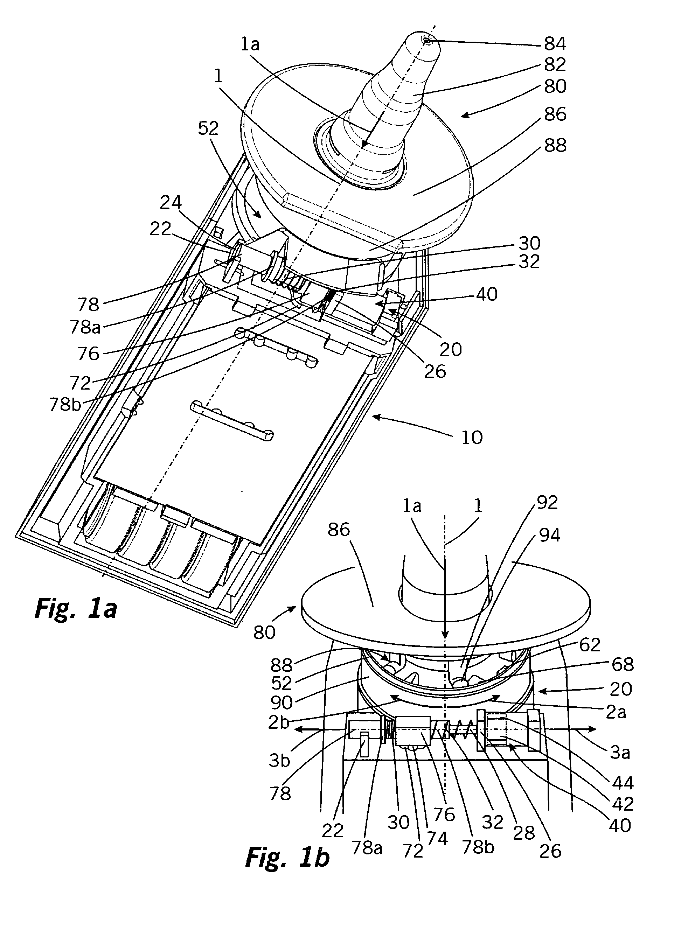

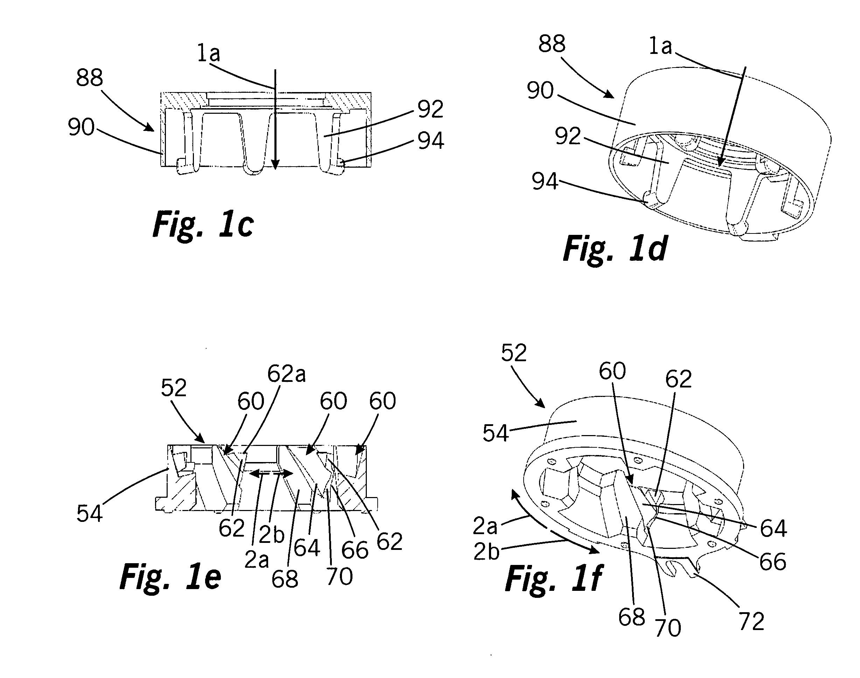

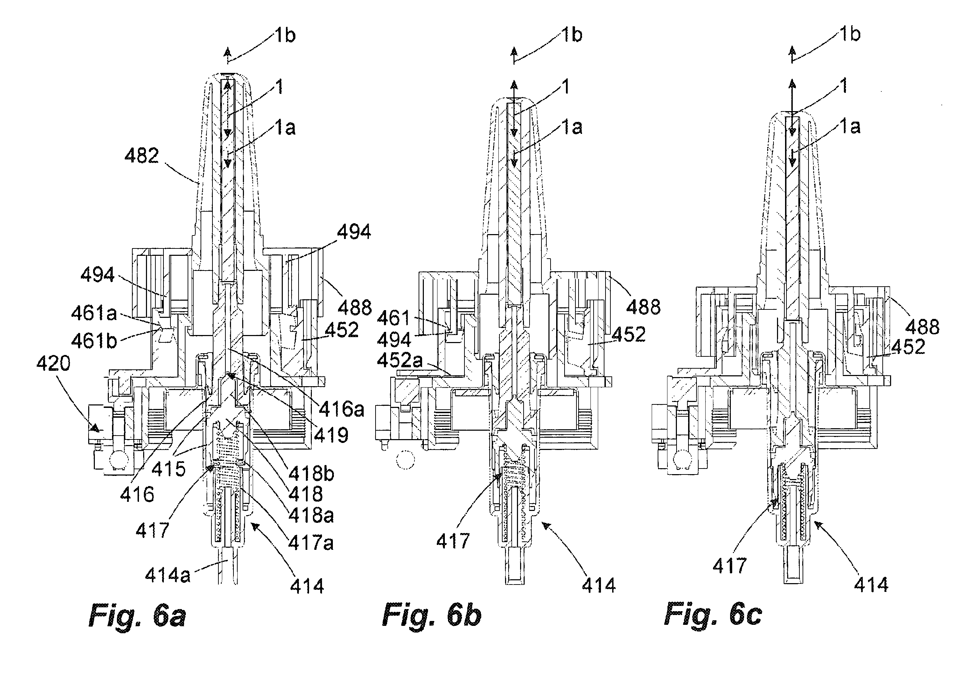

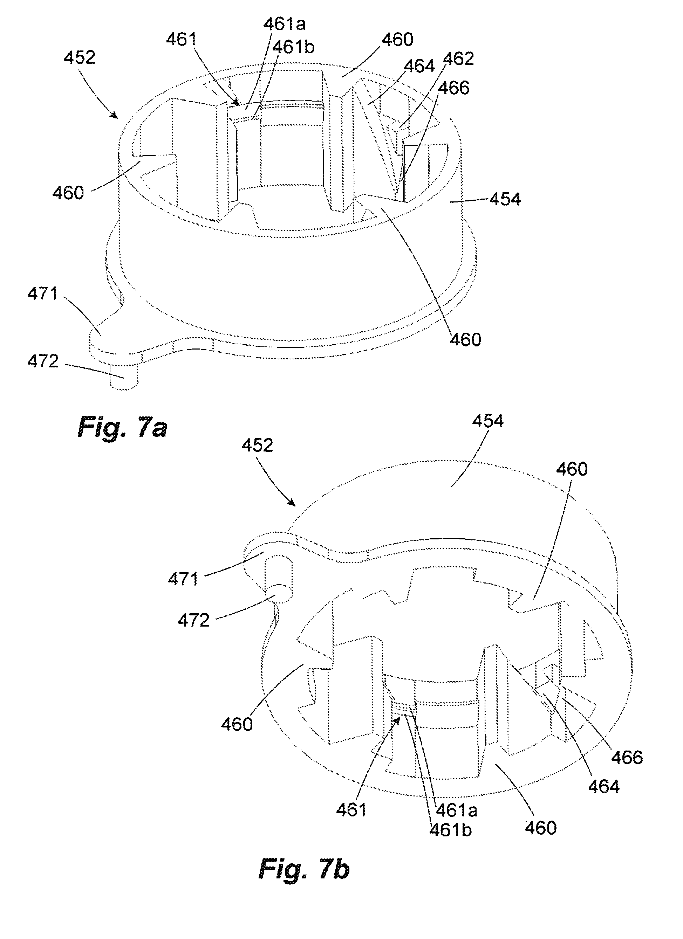

Die Figuren 1 bis 5 zeigen eine Austragvorrichtung bzw. Details davon in verschiedenen Perspektiven. Diese Ausführungsform weist nicht alle der in Kombination miteinander als erfindungsgemäß angesehenen Merkmal auf, dient jedoch als Ausgangspunkt für die erfindungsgemäße Ausgestaltung, die in den Fig. 6 und 7 dargestellt ist.

Dabei zeigt die Figur 1a die Gesamtvorrichtung in einem Zustand mit abgenommenem Elektronikmodul, welches im Betrieb frontseitig angekoppelt ist. Die Figur 1b zeigt die Sperrmechanik der Austragvorrichtung im Detail. Die Figuren 1c bis 1f zeigen geschnittene und perspektivische Ansichten eines Betätigungsgliedes und eines Sperrgliedes der Austragvorrichtung, wobei diese Glieder den Kern der Sperrmechanik darstellen.

Die Darstellungen der Figuren 1a bis 1f sollen der Verdeutlichung der einzelnen Elemente der Austragvorrichtung dienen. Das Zusammenwirken wird in den nachfolgenden Figurengruppen 2 bis 5 erläutert.

Die Austragvorrichtung der Fig. 1a weist ein Gehäuse 10 auf, welches neben einem nicht dargestellten Medienreservoir sowie einer nicht dargestellten Pumpeinrichtung eine Sperrmechanik 20 aufweist. Am oberen Ende schließt sich an das Gehäuse 10 oberhalb der Sperrmechanik 20 eine aus mehreren Bestandteilen bestehende, jedoch in sich starre Applikatorbaugruppe 80 an. Diese weist neben einer Nasenolive 82 mit Auslassöffnung 84 eine Betätigungshandhabe 86 auf, die in einer Betätigungsrichtung 1a gegenüber dem Gehäuse 10 translativ gegen die Federkraft einer nicht dargestellten Pumpenfeder beweglich ist. Diese Verlagerung der Applikatorbaugruppe führt in nicht näher dargestellter Art und Weise zu einer Betätigung der im Gehäuse 10 vorgesehenen Pumpe, deren Pumpkammervolumen hierdurch verringert wird, so dass das in der Pumpkammer zuvor vorhandene Medium durch die Auslassöffnung 84 ausgetragen wird.

Die Besonderheit der Austragvorrichtung liegt in dem Sperrmechanismus 20, der dazu dient, in Abhängigkeit von Randparametern die Bewegung der Applikatorbaugruppe 80 in Betätigungsrichtung 1a zu gestatten oder zu unterbinden. Hierfür umfasst der Sperrmechanismus 20 an der Applikatorbaugruppe 80 unterhalb der Betätigungshandhabe 86 ein etwa ringförmiges Betätigungsglied 88. Dieses Betätigungsglied 88 ist in den Figuren 1c, 1d separat abgebildet und in der Fig. 1b zur Verdeutlichung mit teilweise geschnittener Mantelfläche 90 dargestellt.

Innerhalb der Mantelfläche 90 des Betätigungsgliedes 88 erstrecken sich insgesamt sechs Nockenträger 92 vertikal nach unten, an deren Ende jeweils eine nach außen weisende Nocke 94 vorgesehen ist. Die sechs Nockenträger 92 mit Nocken 94 sind auf einem Kreisbogen gleichmäßig angeordnet und somit voneinander jeweils um 60° beabstandet. Das Betätigungsglied 88 und damit auch die Nocken 94 sind fest mit der Betätigungshandhabe 86 und den anderen Elementen der Applikatorbaugruppe 80 verbunden. Ein Niederdrücken der Betätigungshandhabe 88 führt daher stets ebenfalls zu einem Niederdrücken der Nocken 94. Während die Applikatorbaugruppe 80 in der Betätigungsrichtung 1a gegenüber dem Gehäuse 10 beweglich ist, bestehen keine darüber hinausgehenden Freiheitsgrade. So ist insbesondere die Applikatorbaugruppe 80 gegenüber dem Gehäuse 10 nicht um die Achse 1 der Betätigungsrichtung 1a drehbar, sondern verbleibt stets in einer definierten Winkelstellung zum Gehäuse 10.

Korrespondierend zum Betätigungsglied 88 ist ein Sperrglied 52 vorgesehen. Dieses Sperrglied ist separat in den Figuren 1e und 1f dargestellt und auch in der Darstellung der Fig. 1b zu erkennen.

Dieses Sperrglied 52 ist wie auch das Betätigungsglied 88 in etwa ringförmig ausgebildet und wird von einer äußeren Mantelfläche 54 begrenzt, wobei der Außendurchmesser dieser Mantelfläche 54 geringer als der Innendurchmesser der Mantelfläche 90 des Betätigungsgliedes 88 ist, damit die Mantelflächen 54, 90 ineinander geschoben werden können. Anders als das Betätigungsglied 88 ist das Sperrglied 52 in Betätigungsrichtung 1a nicht beweglich, sondern relativ zum Gehäuse 10 stets auf gleicher Höhe angeordnet. Allerdings ist das Sperrglied 52 in begrenztem Umfang um eine Hauptachse 1, die koaxial zur Betätigungsrichtung 1a ausgerichtet ist, in Richtung 2a, 2b drehbar gelagert. Korrespondierend zu den Nocken 94 am Betätigungsglied 88 sind am Sperrglied 52 insgesamt sechs identisch geformte und nach innen weisende Funktionsabschnitte 60 vorgesehen. Diese Funktionsabschnitte 60 erstrecken sich von der Mantelwandung 54 radial nach innen. Sie weisen an ihrem oberen Ende jeweils einen Sperrabschnitt 62 mit einer etwa ebenen oberen Abschlussfläche 62a auf. Darüber hinaus weisen sie jeweils einen ersten Kulissenspurabschnitt 64 sowie einen zweiten Kulissenspurabschnitt 66 auf. Während der erste Kulissenspurabschnitt zum einen vom Sperrabschnitt 62 und zum anderen von einer Erhebung 68 begrenzt wird und in Richtung 1a leicht radial nach innen geneigt ist, wird der zweite Kulissenspurabschnitt 66 lediglich durch die bezogen auf Fig. 1e rechtsseitige Außenkante des Funktionsabschnitts 60 gebildet. Den Übergang vom ersten Kulissenspurabschnitt 64 in den zweiten Kulissenspurabschnitt 66 bildet eine Stufe 70 an dieser rechtsseitigen Außenkante des Funktionsabschnitts 60.

Wie aus den Figuren 1b und 1f deutlich wird, erstreckt sich von der Mantelwandung 54 eine Führungsgabel 72 radial nach außen. Diese führt einen Zapfen 74, welcher einstückig an einem Hülsenelement 76 angebracht ist. Das Hülsenelement 76 ist schwimmend auf einem Stößel 78 gelagert. Im Sinne dieser Erläuterung werden das Hülsenelement 76 und insbesondere der Stößel 78 als dem Sperrglied 52 zugeordnete Sperrhilfsglieder verstanden.

Anders als das Sperrglied 52 ist der Stößel 78 nicht um die Drehachse 1 drehbar beweglich, sondern lediglich entlang einer Bewegungsachse 3, die tangential zum drehbaren Sperrglied 52 ausgerichtet ist, translativ in die Richtungen 3a, 3b beweglich.

Der Stößel 78 ist zweiseitig gelagert. Zum einen ist am Gehäuse eine Aufnahme 22 mit einer halbkreisförmigen Ausnehmung 24 vorgesehen, in der das bezogen auf die Figur 1b linksseitige Ende des Stößels 78 liegt. Zum anderen ist rechtsseitig des Stößels 78 eine Anschlagsfläche 26 vorgesehen, von der aus sich ein Führungsdorn 28 in Richtung 3b erstreckt, auf den der Stößel 78 mittels einer nicht dargestellten Bohrung im Stößel 78 aufgeschoben ist.

Linksseitig der schwimmend auf dem Stößel 78 gelagerten Hülse 76 ist ein umlaufender Stößelflansch 78a einstückig am Stößel 78 vorgesehen. Zwischen dem Stößelflansch 78a und der Hülse 26 ist eine als Spiralfeder ausgebildete Stößelfeder 30 vorgesehen. Zwischen dem rechtsseitigen Ende der Hülse 76 und der Anschlagsfläche 26 ist eine Freigabefeder 32 vorgesehen. Das durch die Freigabefeder 32 umgebene rechtsseitige Ende 78b des Stößels 78 ist in nicht näher dargestellter Art und Weise permanentmagnetisch ausgebildet. Zum Zusammenwirken mit diesem permanentmagnetischen Ende 78b des Stößels 78 ist rechtsseitig der Anschlagsfläche 26 gehäusefest eine Magneteinheit 40 vorgesehen, die zum einen einen in Fig. 1b gepunktet dargestellten Permanentmagneten 42 aufweist, der den Stößel in die Richtung 3a kraftbeaufschlagt, und die zum anderen eine ebenfalls gepunktet dargestellten Elektromagneten 44 aufweist, der dafür ausgebildet ist, den Stößel 78 bei Bestromung in Richtung des Pfeils 3b kraftzubeaufschlagen.

Die in den Figuren 1a bis 1f dargestellte Ausführungsform gestattet es mittels des Sperrgliedes und des Betätigungsgliedes, in besonders sicherer und energieeffizienter Art und Weise, wahlweise einen Austragvorgang zu blockieren oder zu ermöglichen.

Die Funktionsweise wird anhand der Figurengruppen 2 bis 5 näher erläutert.

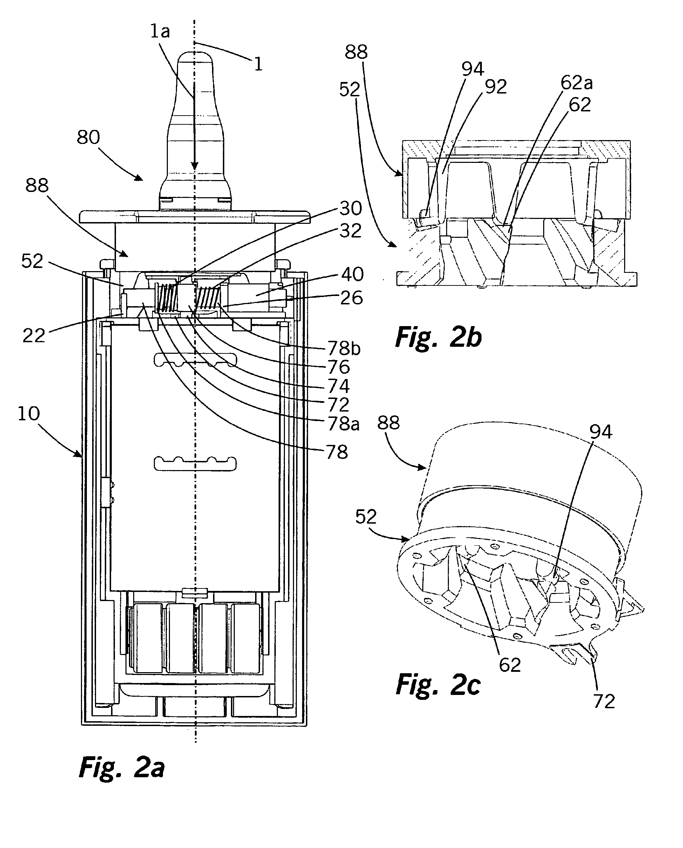

Dabei zeigen die Figuren 2a bis 2c einen Ausgangszustand, in dem ein Austragvorgang blockiert ist. Wie der Figur 2a zu entnehmen ist, liegt in diesem Zustand der Stößel 78 an der Anschlagsfläche 26 an. Trotz der Federkraft der Federn 30,32, die den Stößel 78 in die Richtung 3b kraftbeaufschlagen, löst sich der Stößel 78 von der Anschlagsfläche 26 nicht, da die Permanentmagneten 78b, 42 in der Magneteinheit 40 und am rechtsseitigen Ende des Stößels 78 eine stärkere Kraftbeaufschlagung des Stößels 78 in Richtung 3a bewirken als die Federn 30, 32 in entgegengesetzte Richtung 3b. Die Position des Hülsenelements 76 ergibt sich resultierend aus dem Kräftegleichgewicht an den Federn 30, 32.

Wie aus der zuvor erläuterten Figur 1b deutlich wird, bestimmt die Position des Hülsenelements 76 über den Zapfen 74 und die Führungsgabel 72 zwingend die Drehstellung des Sperrgliedes 52. Aus den Figuren 2b und 2c ist zu entnehmen, welche Drehstellung relativ zu den Nocken 94 beim Zustand der Figur 2a vorliegt. Es ist ersichtlich, dass in dieser Drehstellung die Nocken 94 jeweils direkt oberhalb der Sperrabschnitte 62 angeordnet sind.

Wenn in dem Zustand der Figuren 2a bis 2c eine Betätigung durch den Bediener erfolgt, kann das Betätigungsglied 88 nur in sehr geringem Maße in Richtung 1a verschoben werden. Die Bewegung des Betätigungsgliedes 88 endet, sobald die Nocken 94 auf den obenseitigen Flächen 62a der Sperrabschnitte 62 zum Anliegen kommen. Da das Sperrglied 52 selbst in Richtung 1a nicht beweglich ist, sind ein darüber hinausgehendes Bewegen des Betätigungsgliedes 88 und damit ein Austragen von Medium somit nicht möglich.

Aufgrund der insgesamt sechs Nocken 94, die umlaufend verteilt sind, führt auch eine nicht bestimmungsgemäße, gewalttätige Kraftbeaufschlagung des Betätigungsgliedes 88 orthogonal zur Betätigungsrichtung 1a nicht zu einem Abgleiten aller Nocken 94 von den Flächen 62a der Sperrabschnitte 62. Selbst wenn einseitig ein solches Abgleiten erreicht würde, kann dies niemals für alle Nocken 94 erreicht werden. Der gesperrte Zustand der Austragvorrichtung ist daher vollständig sicher.

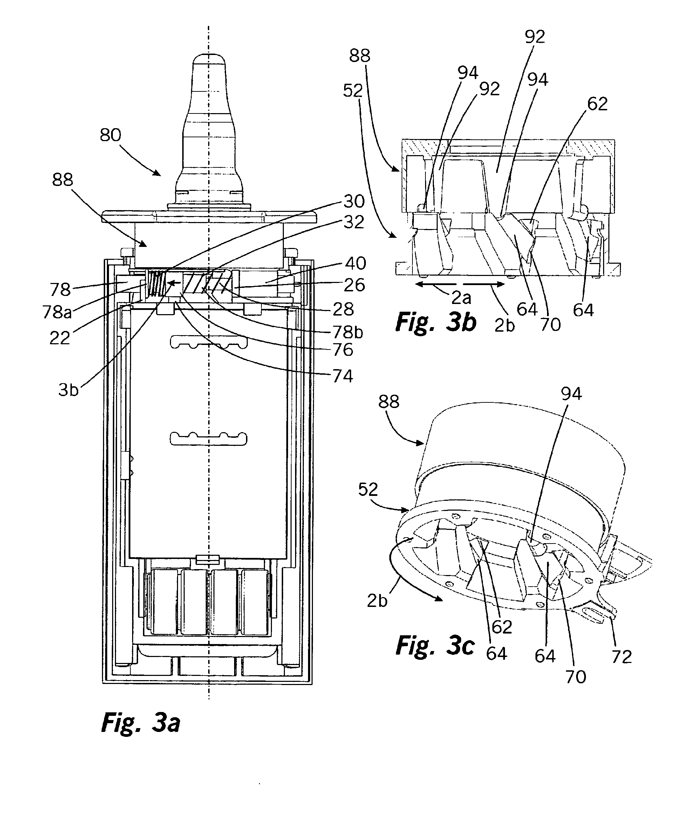

Die Austragvorrichtung ist dafür ausgebildet, dass sie nach einem durch beispielsweise einen Arzt vorgebbaren und in der Elektronik der Austragvorrichtung gespeicherten Zeitintervall nach der vorherigen Benutzung wieder aus ihrer Sperrstellung in ihre Freigabestellung überführt wird. Der Freigabezustand und das Erreichen des Freigabezustands werden nachfolgend anhand der Figuren 3a bis 3c erläutert.

Die Freigabe wird durch Bestromung des Elektromagneten 44 in der Magneteinheit 40 erzielt. Diese Bestromung wird durch ein nicht dargestelltes Steuergerät ausgelöst, welches in Abhängigkeit der genannten Randparameter die Überführung in den Freigabezustand initiiert. Die Bestromung führt zu einer Kraftbeaufschlagung des rechtsseitigen Endes des Stößels 78 in Richtung 3b. Gemeinsam mit der Federkraft der Federn 30, 32 übersteigt die resultierende auf den Stößel 78 in Richtung 3b wirkende Kraft damit die in entgegengesetzte Richtung 3a wirkende Kraft der Permanentmagneten 78b, 42. Demzufolge löst sich der Stößel 78 von der Anschlagsfläche 26 und bewegt sich gespeist von der Federenergie der Feder 30, 32 in Richtung 3b, bis der Stößelflansch 78a an der Aufnahme 22 zum Anschlag kommt.

Die Wirkung der Permanentmagneten 78b, 42 ist örtlich sehr begrenzt, so dass sie schon bei einer geringfügigen Beabstandung des Stößels 78 von der Anschlagsfläche 26 vernachlässigbar klein wird. Während der Bewegung des Stößels 78 muss die Bestromung des Elektromagneten 44 der Magneteinheit 40 daher nicht aufrecht erhalten werden, da bei der entsprechenden Auslegung der Permanentmagneten 78b, 42 und der Federn 30, 32 ein kurzer Impuls ausreicht, um den Stößel 78 bis in die in der Figur 3a dargestellte Stellung zu bringen.

In der dargestellten Endlage des Stößels 78 ergibt sich die Position des Hülsenelements 76 wiederum so, dass die jeweils von den Federn 30 und 32 ausgehende Kraft identisch ist. Aufgrund der Tatsache, dass die rechtsseitige Freigabefeder 32 erheblich härter ist, wird die Hülse 76 vergleichsweise weit in Richtung 3b verschoben. Die Figuren 3b und 3c zeigen die resultierende Relativlage des Betätigungsgliedes 88 und des Sperrgliedes 52. Es ist zu ersehen, dass sich das Sperrglied bezogen auf die Perspektive der Figur 3b in Richtung 2b nach rechts verlagert hat, so dass die Nocken 94 nicht mehr oberhalb der Sperrabschnitte 62 angeordnet sind, sondern oberhalb der Eingangsbereiche der ersten Kulissenspurabschnitte 64. Demzufolge entfällt in dieser Freigabestellung die Limitierung der Bewegungsfreiheit der Nocken 94 nach unten.

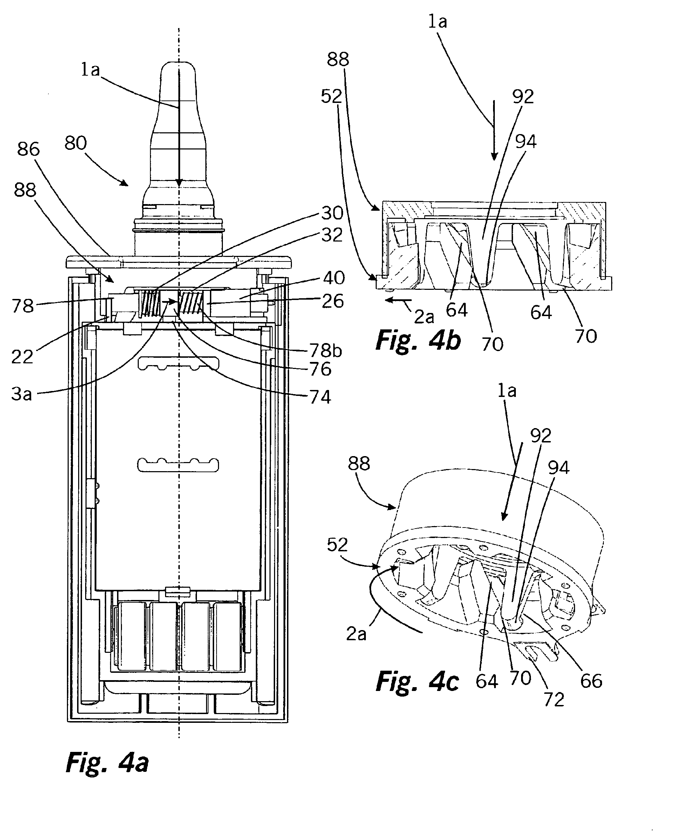

Ausgehend von diesem Freigabezustand der Figuren 3a bis 3c kann daher nun eine Hubbewegung zur Erzeugung eines Medienaustrags erfolgen. Bei der Bewegung des Betätigungsgliedes 88 in Richtung 1a nach unten fahren die Nocken 94 in die ersten Kulissenspurabschnitte 64 ein und verdrehen in Folge der Schrägstellung dieser Kulissenspurabschnitte 64 das Sperrglied 52 bezogen auf die Perspektive der Figur 3b nach links in Richtung 2a. Das Hinabdrücken des Betätigungsgliedes 88, welches mit einem Austragvorgang einhergeht, geht demnach gleichzeitig mit einem Bewegen des Sperrgliedes 52 in Richtung der Sperrstellung einher. Die L-förmige Gestalt der Nockenträger 92 und der Nocken 94 sorgt dafür, dass die Nockenträger 92 während der Bewegung vom Zustand der Fig. 3a in den Zustand der Fig. 4a nicht mit dem Sperrabschnitt 60 kollidieren.

Während der Bewegung des Betätigungsgliedes 88 nach unten in Richtung 1a wird ein elastischer Spannungszustand in den Nockenträgern 92 erzeugt, da die Nocken 94 aufgrund der Formgebung der ersten Führungsspurabschnitte 64 radial zunehmend nach innen ausgelenkt werden. Sobald die Nocken 94 das untere Ende der Führungsspurabschnitte 64 erreicht haben, werden sie über die das Ende bildende Stufe 70 hinübergeschoben, was eine schlagartige Entspannung der gespannten Nockenträger 92 zur Folge hat. Dabei verlagern sich die Nocken 94 wieder radial nach außen.

Die Figuren 4a bis 4c zeigen den dadurch erreichten Zustand. In diesem Zustand, der in etwa den Umkehrpunkt zwischen Hubbewegung und Rückhubbewegung darstellt, ist aufgrund der bis dahin erfolgten Drehbewegung des Sperrgliedes 52 bereits wieder der Zustand erreicht, in dem der Stößel 78 an der Anschlagsfläche 26 anliegt. Er ist daher in der oben beschriebenen Art und Weise in diesem Zustand bereits wieder durch die Permanentmagneten 78b und 42 gehalten.

Der Austragvorgang ist beim Erreichen des Zustandes der Figuren 4a bis 4c bereits abgeschlossen. Ausgehend vom Zustand der Figuren 4a bis 4c erfolgt die Rückhubbewegung, sobald der Bediener die Kraftbeaufschlagung des Betätigungsgliedes 88 nach unten in Richtung 1a entfallen lässt. Sobald dies geschieht, wird die gesamte Applikatorbaugruppe 80 mitsamt dem Betätigungsglied 88 durch die nicht dargestellte Rückstellfeder der Pumpeinrichtung entgegen der Richtung 1a nach oben verlagert. Aufgrund der Tatsache, dass die Nocken 94 über die Stufe 70 hinausbewegt wurden, ist ein entgegengesetzter Bewegungspfad der Nocken 94 entlang der ersten Kulissenspurabschnitte 64 nicht möglich. Stattdessen bewegen sich die Nocken 94 entlang der zweiten Kulissenspurabschnitte 66, also entlang der rechtsseitigen Kante der Funktionsabschnitte 60 nach oben, so dass die Verdrehung des Sperrgliedes 52 bezogen auf die Figuren 4b nach links in Richtung 2a noch fortgesetzt wird. Da eine korrespondierende Bewegung des Stößels 78 in Richtung 3a nicht mehr möglich ist, da der Stößel 78 in dieser Phase bereits an der Anschlagsfläche 26 anliegt, wird durch dieses Weiterdrehen des Sperrgliedes 52 in Richtung 2a lediglich noch das Hülsenelement 76 mitverlagert. Gleichzeitig wird die rechtsseitige Freigabefeder 32 zwischen dem Hülsenelement 76 und der Anschlagsfläche 26 noch weiter gestaucht.

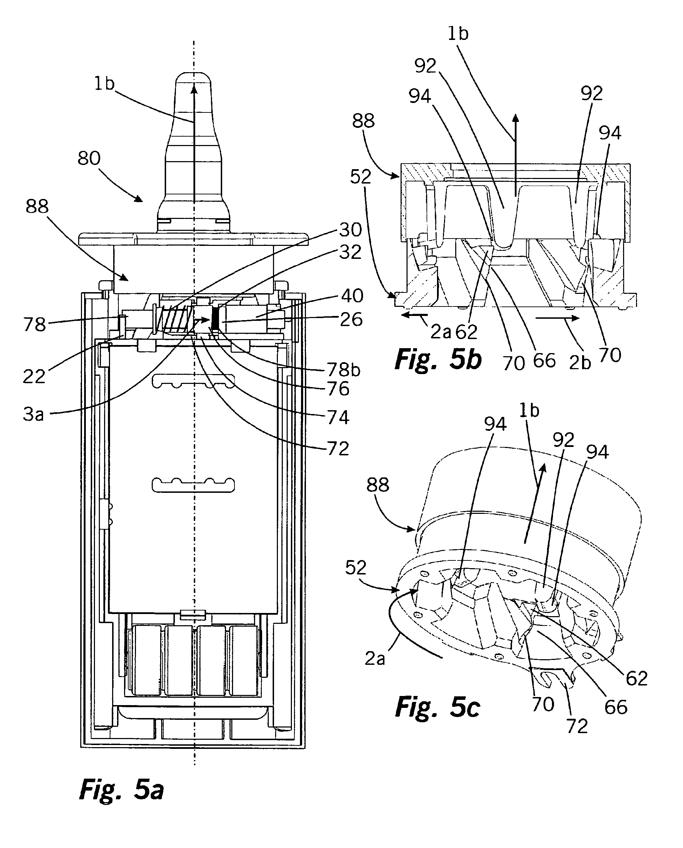

Die Figuren 5a bis 5c zeigen die letzte Phase des Rückhubs. Wie anhand der Figur 5a zu ersehen ist, erreicht die Freigabefeder 32 zwischenzeitlich einen extrem gestauchten Zustand, der erreicht wird, wenn, wie in den Figuren 5b und 5c ersichtlich, die Nocken 94 im Bereich der rechtsseitigen Spitze der Sperrabschnitte 62 an der Außenkante der Funktionsabschnitte entlanggleiten.

In dem Augenblick, in dem die Nocken 94 außer Eingriff mit den Sperrabschnitten 62 gelangen und damit den zweiten Führungsspurabschnitt 66 verlassen, drückt die extrem gestauchte Freigabefeder 32 die Hülse schlagartig nach links, was in der Perspektive der Figuren 5b und 5c ein Verdrehen des Sperrgliedes 52 nach rechts entgegen der Richtung 2a zur Folge hat. Diese Bewegung endet, sobald an den Federn 30, 32 wieder ein Kräftegleichgewicht herrscht. Durch die Bewegung werden die Nocken 94 relativ zu den Sperrabschnitten 62 wieder so verlagert, dass sie ihre Sperrlage oberhalb der Sperrabschnitte 62 einnehmen. Damit ist wieder der Zustand der Figuren 2a bis 2c erreicht.

Die beschriebene Gestaltung ermöglicht einen Sperrmechanismus für eine Austragvorrichtung, bei dem für die Bewegung des entsprechenden Sperrgliedes keinerlei Energiequelle in der Austragvorrichtung vorgesehen sein muss, da die Überführung in die Sperrstellung unmittelbar durch die vom Bediener eingebrachte Energie gespeist wird und da die Überführung in die Freigabestellung mittelbar durch die vom Bediener eingebrachte Energie, zwischengespeichert in den Federn 30, 32, gespeist wird. Nur zum Auslösen der Bewegung in die Freigabestellung ist ein Auslösemittel, beispielsweise in Form eines Elektromagneten 44, vorgesehen. Die Aufgabe dieses Elektromagneten 44 ist jedoch nicht die Aufbringung der zur Verlagerung des Sperrgliedes erforderlichen Energie, sondern die lediglich kurzzeitige Bewirkung einer geringen zusätzlichen Kraft auf den Stößel, die gemeinsam mit der Federkraft der Federn 30, 32 ein Ablösen des Stößels von der Anschlagsfläche 26 bewirkt.

Die Fig. 6a bis 6b zeigen eine erfindungsgemäße Ausführungsform einer erfindungsgemäßen Austragvorrichtung. Dabei zeigen die Fig. 6a bis 6c Teilansichten der Austragvorrichtung, die hinsichtlich der meisten Bestandteile mit der Ausführungsform der Fig. 1 bis 5 übereinstimmt. Die Austragvorrichtung weist eine Sperrmechanik 420 auf, die mit der Sperrmechanik 20 der Fig. 1 bis 5 übereinstimmen kann.
Dargestellt ist auch eine innerhalb des Gehäuses der Austragvorrichtung angeordnete Pumpe 414 mit einer Pumpkammer 415. Bei dieser Pumpe 414 handelt es sich um eine Kolbenpumpe, deren Kolben 416 zum Zwecke der Volumenveränderung der Pumpkammer 415 gemeinsam mit dem Betätigungsglied 488, der Nasenolive 482 und einer nicht dargestellten Betätigungshandhabe in Richtung des Pfeils 1 manuell verlagerbar ist. Die Besonderheit dieser Pumpe liegt in der Gestaltung des Einlassventils 417. Dieses Einlassventil 417 umfasst eine an einem Ventilkörper 418 vorgesehene umlaufende und nach innen gewandte Ventillippe 418a am unteren Ende des Ventilkörpers 418 und korrespondierend dazu einen pumpengehäusefesten Einlassstutzen 417a, dessen Außenumfang an den Innenumfang der Ventillippe 418a angepasst ist.

Die Funktionsweise dieser Pumpe 414 ist die folgende: Bei einer Hubbewegung des Betätigungsgliedes 488 in Richtung 1a werden zunächst auch der Kolben 415 und der Ventilkörper 418 verlagert. Sobald dabei die Ventillippe 418a in Kontakt mit dem Einlassstutzen 417a gelangt und auf diesen aufgeschoben wird, ist die Pumpkammer 415 in Richtung eines Einlasskanals 414a abgeschlossen. Ein Weiterbewegen des Kolbens 416 führt dann zu einer druckbedingten Verlagerung des Ventilkörpers 418 in Richtung 1a, wobei diese Bewegung schneller als die Verlagerung des Kolbens 416 in gleiche Richtung abläuft. Daraus resultierend öffnet ein Pumpenauslassventil 419, welches durch eine konische Spitze 418b des Ventilkörpers 418 und einen Auslasskanal 416a im Kolben 416 gebildet ist. Der Austragvorgang beginnt. Wenn nachfolgend die manuelle Kraftbeaufschlagung des Betätigungsgliedes 488 entfällt, werden der Kolben 416 und der Ventilkörper 418 durch die Pumpenfeder in Rückhubrichtung 1 b verlagert, wobei dies zunächst keine Neubefüllung der Pumpkammer 415 bewirkt, da die Ventillippe 418a für einen großen Teil des Rückhubweges noch am Einlassstutzen 417a anliegt. Stattdessen wird ein Unterdruck in der Pumpkammer 415 erzeugt. Erst wenn sich die Ventillippe 418a vom Einlassstutzen 417a löst und damit das Einlassventil 417 öffnet, führt dieser Unterdruck zu einem schlagartigen Einsaugen von Medium durch den Einlasskanal 414a. Diese Trennung von Ventil 418a und Einlassstutzen 417a findet erst gegen Ende des Rückhubs statt, sobald das Betätigungsglied 488 und das Sperrglied 452 außer Eingriff gelangen.

Die Verwendung einer solchen Pumpe 414, die über einen erheblichen Teil des Rückhubs die Pumpkammer 415 nicht neu befüllt und dies erst ab Erreichen einer Befüllungszwischenstellung des Kolbens tut, ergibt sich im Zusammenhang mit den Darstellungen der Fig. 7a und 7b. Diese Figuren zeigen in verschiedener Ansicht den Sperrring 452 der Austragvorrichtung gemäß der Fig. 11a. Dieser Sperrring 452 entspricht bezüglich der meisten Merkmale dem Sperrring, der in den Fig. 1e und 1f dargestellt ist. Er weist eine Mantelwandung 454 auf, von der aus sich radial nach außen ein Fortsatz 471 erstreckt, an dem ein Führungsstift 472 vorgesehen ist, der hinsichtlich seines Zwecks mit der Führungsgabel 72 der Ausführungsform der Fig. 1e und 1f übereinstimmt. Abweichend von der Ausgestaltung der Fig. 1e und 1f sind nicht insgesamt sechs Funktionsabschnitte, sondern stattdessen nur drei Funktionsabschnitte 460 mit Kulissenspurabschnitten 464, 466 an der Innenseite der Mantelwandung 454 angeformt. Zwischen diesen drei Funktionsabschnitten 460 sind jeweils Sperrstufen 461 angeordnet, die an ihrer nach oben gewandten Seite eine Schräge 461 a aufweisen und die an ihrer Unterseite eine im Wesentlichen radial ausgerichtete Sperrkante 461 b aufweisen.

Die Wirkung dieser Sperrstufen 461 ist die folgende: Wenn das Betätigungsglied 488 aus einer nicht gesperrten Ausgangslage gemäß Fig. 6a in Richtung des Pfeils 1a hinabgedrückt wird, führt dies in der zu den vorangegangenen Ausführungsformen beschriebenen Art und Weise zunächst zu einem Verdrehen des Sperrrings 452 und gleichzeitig zum Beginn eines Medienaustrags. Sobald die Nocken 494 des Betätigungsgliedes 488 in den Bereich der Sperrstufen 461 gelangen, werden sie durch die Schrägen 461 a radial nach innen ausgelenkt und springen nach Überwindung der Sperrstufen 461 im Bereich der Sperrkanten 461 b zurück in ihren unausgelenkten Zustand. Wenn dieser Zustand erreicht ist, ist eine Rückführung des Betätigungsgliedes 488 in die zuvor eingenommene Ausgangsstellung zunächst nicht mehr möglich, da die Nocken 494 nicht in Rückhubrichtung 1b über die Rückführstufen 461 hinübergeführt werden können, wie aus Fig. 6b ersichtlich ist. Demzufolge besteht die einzige Möglichkeit zur Rücküberführung des Betätigungsglieds 488 in seine unbetätigte Ausgangslage darin, dass die begonnene Hubbewegung in Richtung 1a zu Ende geführt wird, so dass nach Erreichen der Endlage gemäß Fig. 6c in der oben bereits beschriebenen Art und Weise eine Rückführung der Nocken 494 entlang der zweiten Kulissenspurabschnitte 466 erfolgt. Dies bringt allerdings in oben beschriebener Art zwingend eine Wiederherstellung des Sperrzustandes mit sich.

Diese Gestaltung, die nach Erreichen der Zwischenlage der Fig. 6b eine Rücküberführung in den Ausgangszustand der Fig. 6a nur über Durchlaufen der Endstellung gemäß 6c gestattet, verhindert in Verbindung mit der oben beschriebenen Gestaltung der Pumpe 414, dass ein Benutzer die Austragvorrichtung missbräuchlich verwendet, indem er wiederholte Teilhubbewegungen durchführt, bei denen die Nocken 494 zwischen der Sperrkante 461 b und der Unterkante 452a des Sperrgliedes 452 hin- und herbewegt werden. Eine solche Betätigung führt nicht zum Erfolg, da hierbei aufgrund der Gestaltung des Einlassventils 417 der Pumpe 414 keine Wiederbefüllung der Pumpkammer 415 erzielt werden kann. Wie oben bereits erwähnt wurde, wird die Pumpkammer 415 erst wieder dann befüllt, wenn die Rückhubbewegung in Richtung 1b fast abgeschlossen oder abgeschlossen ist. Die Bewegung des Betätigungsgliedes 488 in Richtung 1b, bis die Nocken 494 an den Sperrkanten 461 b anschlagen, reicht hierfür nicht aus. Erst wenn die Nocken entlang der zweiten Kulissenspurabschnitten 466 in Richtung 1b gegenüber dem Sperrglied 452 verfahren werden, wird diese Neubefüllung der Pumpkammer 415 erzielt. Da hierdurch jedoch auch zwingend der Sperrzustand hergestellt wird, ist ein Missbrauch nicht möglich.

Bei einer nicht dargestellten Variante zur Ausführungsform der Fig. 6a bis 7b sind statt der jeweils nur einen Sperrstufe 461 je Nocke 494 eine Vielzahl von solchen Stufen hintereinander entlang der Hubbewegung 1a vorgesehen, so dass ein Rückhub fast vollständig verhindert wird, da im Zuge der Hubbewegung diese eine Rastleiter bildenden Sperrstufen jeweils nach Überwindung in Hubrichtung 1a eine Rückstellung in Rückhubrichtung 1 b verhindern.

## Patentansprüche

1. Austragvorrichtung für Medien mit
- einem Gehäuse,
- ein Austragmittel mit einer Pumpe (414) mit einer volumenveränderlichen Pumpkammer (415),
- einem gegenüber dem Gehäuse manuell bewegbaren Betätigungsglied (488), das zum Zwecke der Betätigung des Austragmittels (414) aus einer unbetätigten Ausgangslage in Richtung einer Betätigungsrichtung (1a) in eine betätigte Endlage überführbar ist,
- einem Sperrglied (452), welches zwischen einer Sperrstellung, in der es die Verlagerung des Betätigungsgliedes (488) in die Endlage verhindert, und einer Freigabestellung, in der es die Verlagerung des Betätigungsgliedes (488) in die Endlage ermöglicht, gegenüber dem Gehäuse verlagerbar ist, und
- eine Rückführsperre (461) vorgesehen ist, die nach Erreichen einer definierten Sperrzwischenstellung bei einer Überführung des Betätigungsgliedes (488) aus der Ausgangslage in Richtung der Endlage eine Rückführung des Betätigungsgliedes (488) in die Ausgangslage solange verhindert, bis das Betätigungsglied (488) in die Endlage überführt wird,
**dadurch gekennzeichnet, dass**
die Pumpe (414) ein Einlassventil (417) aufweist und derart gestaltet ist, dass im Rahmen eines Rückhubs erst ab einer definierten Befüllungszwischenstellung das Einlassventil (417) öffnet und eine Befüllung der Pumpkammer (415) erfolgt, wobei diese Befüllungszwischenstellung derart angeordnet ist, dass sie während des Rückhubs erst dann erreicht wird, wenn sich das Betätigungsglied (488) in einer Lage zwischen der Sperrzwischenstellung und der Ausgangslage befindet.

2. Austragvorrichtung nach einem der Ansprüche 1,
**dadurch gekennzeichnet, dass**
die Rückführsperre (461) Rastmittel (461 b, 494) aufweist, wobei ein erster Bestandteil (461b) der Rastmittel (461b, 494) in Betätigungsrichtung (1a) ortsfest zum Gehäuse angeordnet ist und wobei ein zweiter Bestandteil (494) der Rastmittel, der zum Zusammenwirken mit dem ersten Bestandteil (461b) ausgebildet ist, in Betätigungsrichtung (1a) ortsfest zum Betätigungsglied (488) angeordnet ist.

3. Austragvorrichtung nach Anspruch 1 oder 2,
**dadurch gekennzeichnet, dass**
- ein Federmittel vorgesehen ist, durch welches das Sperrglied (452) in Richtung seiner Freigabestellung kraftbeaufschlagt wird und
- das Betätigungsglied (488) und das Sperrglied (452) derart miteinander wirkgekoppelt sind, dass in der Freigabestellung eine Hubbewegung des Betätigungsgliedes (488) aus der unbetätigten Ausgangslage in die betätigte Endlage und/oder eine nachfolgende Rückhubbewegung aus der betätigten Endlage in die Ausgangslage mittels einer Übertragungsmechanik (464, 466, 494) eine Energieeinspeisung in das erste Federmittel bewirkt.

4. Austragvorrichtung nach Anspruch 3,
**dadurch gekennzeichnet, dass**
das Betätigungsglied (488) und das Sperrglied (452) über eine Kulissenführung (464, 466, 494) miteinander gekoppelt sind, mittels derer die Hubbewegung und/oder die Rückhubbewegung des Betätigungsgliedes (488) die Verlagerung des Sperrgliedes (452) bewirkt.

5. Austragvorrichtung nach Anspruch 4,
**dadurch gekennzeichnet, dass**
die Kulissenführung (464, 466, 494) mindestens eine Kulissenspur (464, 466) am Sperrglied (452) und mindestens eine Nocke (492) zum Eingriff in die Kulissenspur (464, 466) am Betätigungsglied (488) aufweist, wobei die Kulissenführung (94464, 466, 494) derart ausgebildet ist, dass sie einen ersten Kulissenspurabschnitt (464) aufweist, in den die Nocke (494) bei der Hubbewegung einfährt, und/oder einen zweiten Kulissenspurabschnitt (466) aufweist, in den die Nocke (494) gegen Ende der Hubbewegung oder beim Übergang in die Rückhubbewegung einfährt.

6. Austragvorrichtung nach einem der vorstehenden Ansprüche,
**dadurch gekennzeichnet, dass**
eine Hubbewegung des Betätigungsgliedes (488) in der Sperrstellung des Sperrgliedes (452) dadurch zumindest abschnittsweise unterbunden wird, dass mindestens ein sperrgliedseitiger Sperrabschnitt (462) den Hubbewegungspfad von mindestens einem betätigungsgliedseitigen Sperrabschnitt (494) versperrt, wobei der betätigungsgliedseitige Sperrabschnitt (494) vorzugsweise mit der der betätigungsgliedseitigen Nocke (494) der Kulissenführung (494, 464, 466) identisch ist.

7. Austragvorrichtung nach einem der vorstehenden Ansprüche,
**dadurch gekennzeichnet, dass**
eine Steueranordnung vorgesehen ist, die dafür ausgebildet ist,
- in einem Blockierzustand die Bewegung des Sperrgliedes (452) gegenüber dem Gehäuse zu blockieren und
- durch eine mittels eines elektrisches Signal erzielbare Auslösung die Verlagerung des Sperrgliedes (452) aus der Sperrstellung in die Freigabestellung zu ermöglichen.

8. Austragvorrichtung nach Anspruch 7,
**dadurch gekennzeichnet, dass**
die Steueranordnung derart ausgebildet ist, dass sie bei einer Verlagerung des Sperrgliedes (452) in die Sperrstellung automatisch in den Blockierzustand überführt wird.

9. Austragvorrichtung nach einem der Ansprüche 7 oder 8,
**dadurch gekennzeichnet, dass**
ein Riegelglied vorgesehen ist, welches im Blockierzustand der Steueranordnung eine Verlagerung des Sperrgliedes (452552) oder eines mit dem Sperrglied wirkverbundenen Sperrhilfsgliedes mechanisch blockiert.

10. Austragvorrichtung nach einem der Ansprüche 7 bis 9,
**dadurch gekennzeichnet, dass**
im Blockierzustand der Steueranordnung das Sperrglied oder ein mit dem Sperrglied (452) wirkverbundenes Sperrhilfsglied durch einen Permanentmagneten in einer Lage gehalten wird, aus der resultierend das Sperrglied (452) in seiner Sperrstellung angeordnet ist, oder
- das Riegelglied oder ein mit dem Riegelglied wirkverbundenes Riegelhilfsglied durch einen Permanentmagneten in einer Lage gehalten wird, aus der die mechanische Blockierung des Sperrgliedes (452) durch das Riegelglied resultiert.

11. Austragvorrichtung nach Anspruch 10,
**dadurch gekennzeichnet, dass**
die durch den Permanentmagneten (446) in der Sperrstellung des Sperrgliedes (452) auf das Sperrglied (452), das Sperrhilfsglied, das Riegelglied und/oder das Riegelhilfsglied ausgeübte Kraft größer ist als die von Federmitteln der Austragvorrichtung in der Sperrstellung des Sperrgliedes in entgegengesetzte Richtung auf das Sperrglied, das Sperrhilfsglied, das Riegelglied bzw. das Riegelhilfsglied wirkende Kraft.

12. Austragvorrichtung nach einem der Ansprüche 7 bis 11,
**dadurch gekennzeichnet, dass**
zur Erzielung der Auslösung ein elektrisch ansteuerbarer Aktuator, vorzugsweise ein Elektromagnet vorgesehen ist, durch den das Sperrglied oder ein mit dem Sperrglied wirkverbundenes Sperrhilfsglied kraftbeaufschlagt werden kann, so dass resultierend das Sperrglied in Richtung seiner Freigabestellung verlagert wird, oder ein Riegelglied oder ein mit dem Riegelglied wirkverbundenes Riegelhilfsglied in eine Richtung kraftbeaufschlagt wird, die zu einem Lösen der mechanischen Blockierung des Sperrgliedes (452) durch das Riegelglied und damit zu einer Verlagerung des Sperrgliedes (452) in Richtung seiner Freigabestellung führt.

13. Austragvorrichtung nach einem der vorstehenden Ansprüche,
**dadurch gekennzeichnet, dass**
das Sperrglied (452) zur Überführung aus der Sperrstellung in die Freigabestellung um eine in Betätigungsrichtung (1a) ausgerichtete Drehachse (1) drehbar ausgebildet ist und über seinen Umfang verteilt mindestens drei Sperrabschnitte (462) aufweist, die in der Sperrstellung des Sperrgliedes (452) derart angeordnet sind, dass eine Hubbewegung des Betätigungsgliedes (488) verhindert wird.

14. Austragvorrichtung nach Anspruch 13,
**dadurch gekennzeichnet, dass**
das Sperrglied (452) das Betätigungsglied (488) umlaufend umgibt und die Drehachse (1) des Sperrgliedes (452) parallel zur Betätigungsrichtung (1a) und vorzugsweise koaxial zu einer Mittelachse (1) der Austragvorrichtung ausgerichtet ist.

## Claims

1. A discharge device for media having
- a housing,
- a discharge means including a pump (414) having a volume variable pump chamber (415),
- an operating member (488) manually movable relative to the housing and which for operating a discharge means (414) can be transferred from an unoperated starting position in the direction of an operating direction (1a) into an operated end position,
- a locking member (452), which is displaceable between a locked position in which it prevents the displacement of the operating member (488) into the end position, and a release position in which it allows the displacement of the operating member (488) into the end position, with respect to the housing, and
- a return lock (461) is provided and which after reaching a defined intermediate locking position during a transition of the operating member (488) from the starting position in the direction of the end position prevent a return of the operating member (488) to the starting position as long as said operating member (488) will be transferred to the end position,
**characterized in that**
the pump (414) has an inlet valve (417) and is configured such that during a return stroke until in a defined intermediate filling position the inlet valve (417) will not open and a filling of the pump chamber (415) is not effected, wherein said intermediate filling position is arranged such, that during the return stroke it is reached not earlier than when the operating member (488) is positioned at a location between the intermediate locking position and the starting position.

2. The discharge device according to claim 1, **characterized in that** the return lock (461) has detent means (461b, 494), a first component (461b) of the detent means (461b, 494) being disposed fixedly relative to the housing in an operating direction (1a) and a second component (494) of the detent means, which is constructed for co-operation with the first component (461 b), being disposed fixedly relative to the operating member (488) in the operating direction (1a).

3. The discharge device according to claim 1 or 2, **characterized in that**
- a spring means is provided which applies a force to locking member (452) in the direction of its release position, and
- the operating member (488) and the locking member (452) are so operatively coupled together that in the release position a stroke movement of the operating member (488) from the unoperated starting position into the operated end position and/or a subsequent return stroke movement from the operated end position into the starting position is brought about by means of a transmission mechanism (466, 494) brings about a supply of energy into the first spring means.

4. The discharge device according to claim 3, **characterized in that** the operating member (488) and the locking member (452) are coupled together by means of a connecting link guide (464, 466, 494), by means of which the stroke movement and/or return stroke movement of the operating member (488) brings about the displacement of the locking member (452).

5. The discharge device according to claim 4, **characterized in that** the connecting link guide (464, 466, 494) has at least one connecting link groove (464, 466) on locking member (452) and at least one cam (492) for engagement in the connecting link groove (464, 466) on operating member (488), the connecting link guide (94, 464, 466, 494) being constructed in such a way that it has a first connecting link groove section (464) into which is introduced the cam (494) during the stroke movement, and/or a second connecting link groove section (466) into which the cam (494) can be introduced towards the end of the stroke movement or on passing into the return stroke movement.

6. The discharge device according to any of the preceding claims, **characterized in that**
a stroke movement of the operating member (488) in the locked position of the locking member (452) is at least sectorwise prevented **in that** at least one locking memberside locking section (462) blocks the stroke movement path of at least one operating member-side locking section (494) and said operating member-side locking section (494) is preferably identical with the operating member-side cam (494) of the connecting link guide (464, 466, 494).

7. The discharge device according to any of the preceding claims, **characterized in that**
a control arrangement is provided and is constructed so as to
- block the movement of the locking member (452) relative to the housing in a blocking state, and
- through a triggering action attainable by means of an electric signal allows the displacement of the locking member (452) from the locked position into the release position.

8. The discharge device according to claim 7, **characterized in that**
the control arrangement is constructed in such a way that on displacement of the locking member (452) into the locked position it is automatically transferred into the blocking state.

9. The discharge device according to claim 7 or 8, **characterized in that**
a bolt member is provided, which in the blocking state of the control arrangement mechanically blocks a displacement of the locking member (452, 552) or a locking auxiliary member operatively connected to the locking member.

10. The discharge device according to any of the claims 7 to 9,
**characterized in that**
in the blocking state of the control arrangement the locking member or a locking auxiliary member operatively connected to the locking member (452) is held by a permanent magnet in a position leading to the locking member (452) being placed in its locked position, or
- the bolt member or a bolt auxiliary member operatively connected to the bolt member is held by a permanent magnet in a position leading to the mechanical blocking of the locking member (452) by the bolt member.

11. The discharge device according to claim 10,
**characterized in that**
the force exerted by the permanent magnet (446) in the locked position of locking member (452) on the locking member (452), locking auxiliary member, bolt member and/or bolt auxiliary member is higher than the force applied by spring means of the discharge device in the locked position of the locking member and in the opposite direction on said locking member, said locking auxiliary member, said bolt member or said bolt auxiliary member.

12. The discharge device according to any of the claims 7 to 11,
**characterized in that**
for bringing about the triggering action an electrically controllable actuator, preferably an electromagnet, is provided, through which the locking member or a locking auxiliary member operatively connected to the locking member can be force-actuated, so that the locking member is displaced in the direction of its release position or a bolt member or a bolt auxiliary member operatively connected to the bolt member is force-actuated in a direction leading to an elimination of the mechanical blocking of the locking member (452) by the bolt member and therefore to a displacement of the locking member (452) towards its release position.

13. The discharge device according to any of the preceding claims, **characterized in that**
for transfer from the locked position into the release position, the locking member (452) is constructed to rotate about a rotation axis (1) oriented in the operating direction (1a) and has distributed over its circumference at least three locking sections (462), which are so positioned in the locked position of the locking member (452) that a stroke movement of the operating member (488) is prevented.

14. The discharge device according to claim 13,
**characterized in that**
the locking member (452) surrounds in all-round manner the operating member (488) and the rotation axis (1) of the locking member (452) is oriented parallel to the operating direction (1a) and preferably coaxially to a centre axis (1) of the discharge device.

## Revendications

1. Dispositif de distribution pour des médias, avec
- un boîtier,
- un moyen de distribution avec une pompe (414) avec une chambre de pompe à volume variable (415),
- un organe d'actionnement (488) déplaçable manuellement par rapport au boîtier, qui, en vue de l'actionnement du moyen de distribution (414), peut être transféré d'une position initiale non actionnée dans la direction d'une direction d'actionnement (1a) à une position finale actionnée,
- un organe de blocage (452), qui est déplaçable entre une position de blocage, dans laquelle il empêche le déplacement de l'organe d'actionnement (488) dans la position finale, et une position de libération, dans laquelle il permet le déplacement de l'organe d'actionnement (488) dans la position finale, et
- il est prévu un blocage de retour (461) qui, après qu'il ait atteint une position intermédiaire de blocage définie lors d'un transfert de l'organe d'actionnement (488) de la position initiale en direction de la position finale, empêche un retour de l'organe d'actionnement (488) dans la position initiale jusqu'à ce que l'organe d'actionnement (488) soit transféré dans la position finale, **caractérisé en ce que** la pompe (414) présente une soupape d'entrée (417) et est configurée de telle manière que, dans le cadre d'une course de retour, la soupape d'entrée (417) ne s'ouvre et n'opère un remplissage de la chambre de pompe (415) qu'à partir d'une position intermédiaire de remplissage déterminée, dans lequel cette position intermédiaire de remplissage est disposée de telle manière qu'elle ne soit atteinte pendant la course de retour que lorsque l'organe d'actionnement (488) se trouve dans une position située entre la position intermédiaire de blocage et la position initiale.

2. Dispositif de distribution selon la revendication 1, **caractérisé en ce que** le blocage de retour (461) présente des moyens d'encliquetage (461b, 494), dans lequel un premier composant (461b) des moyens d'encliquetage (461b, 494) est disposé solidairement au boîtier dans la direction d'actionnement (1a) et dans lequel un deuxième composant (494) des moyens d'encliquetage, qui est configuré pour coopérer avec le premier composant (461b), est disposé solidairement à l'organe d'actionnement (488) dans la direction d'actionnement (1a).

3. Dispositif de distribution selon la revendication 1 ou 2, **caractérisé en ce que**
- il est prévu un moyen de ressort, par lequel l'organe de blocage (452) est soumis à une force dans la direction de sa position de libération, et
- l'organe d'actionnement (488) et l'organe de blocage (452) sont activement couplés l'un à l'autre, de telle manière que, dans la position de libération, un mouvement de course de l'organe d'actionnement (488) de la position initiale non actionnée à la position finale actionnée et/ou un mouvement de course de retour suivant de la position finale actionnée à la position initiale provoque une accumulation d'énergie dans le premier moyen de ressort au moyen d'un mécanisme de transmission (464, 466, 494).

4. Dispositif de distribution selon la revendication 3, **caractérisé en ce que** l'organe d'actionnement (488) et l'organe de blocage (452) sont couplés l'un à l'autre au moyen d'un guidage à coulisse (464, 466, 494), au moyen duquel le mouvement de course et/ou le mouvement de course de retour de l'organe d'actionnement (488) provoque le déplacement de l'organe de blocage (452).

5. Dispositif de distribution selon la revendication 4, **caractérisé en ce que** le guidage à coulisse (464, 466, 494) présente au moins une voie de coulisse (464, 466) sur l'organe de blocage (452) et au moins une came (492) à engager dans la voie de coulisse (464, 466) sur l'organe d'actionnement (488), dans lequel le guidage à coulisse (464, 466, 494) est configuré de façon à présenter une première portion de voie de coulisse (464), dans laquelle la came (494) s'engage lors du mouvement de course, et/ou présente une deuxième portion de voie de coulisse (466), dans laquelle la came (494) s'engage vers la fin du mouvement de course ou lors du passage au mouvement de course de retour.

6. Dispositif de distribution selon l'une quelconque des revendications précédentes, **caractérisé en ce qu'**un mouvement de course de l'organe d'actionnement (488) dans la position de blocage de l'organe de blocage (452) est empêché au moins par segments du fait qu'au moins un segment de blocage (462) côté organe de blocage barre le chemin du mouvement de course d'au moins un segment de blocage (494) côté organe d'actionnement, dans lequel le segment de blocage (494) côté organe d'actionnement est de préférence identique à celui de la came (494) côté organe d'actionnement du guidage à coulisse (494, 464, 466).

7. Dispositif de distribution selon l'une quelconque des revendications précédentes, **caractérisé en ce qu'**il est prévu un dispositif de commande, qui est conçu pour
- dans un état de blocage, bloquer le mouvement de l'organe de blocage (452) par rapport au boîtier, et
- permettre le déplacement de l'organe de blocage (452) de la position de blocage à la position de libération par un déclenchement réalisable au moyen d'un signal électrique.

8. Dispositif de distribution selon la revendication 7, **caractérisé en ce que** le dispositif de commande est conçu de telle manière qu'il soit transféré automatiquement dans l'état de blocage lors d'un déplacement de l'organe de blocage (452) dans la position de blocage.

9. Dispositif de distribution selon l'une des revendications 7 ou 8, **caractérisé en ce qu'**il est prévu un organe de verrouillage qui, dans l'état de blocage du dispositif de commande, bloque mécaniquement un déplacement de l'organe de blocage (452, 552) ou d'un organe auxiliaire de blocage relié opérativement à l'organe de blocage.

10. Dispositif de distribution selon l'une quelconque des revendications 7 à 9, **caractérisé en ce que**, dans l'état de blocage du dispositif de commande,
- l'organe de blocage ou un organe auxiliaire de blocage relié opérativement à l'organe de blocage (452) est maintenu par un aimant permanent dans une position, de laquelle il résulte que l'organe de blocage (452) est disposé dans sa position de blocage, ou
- l'organe de verrouillage ou un organe auxiliaire de verrouillage relié opérativement à l'organe de verrouillage est maintenu par un aimant permanent dans une position, de laquelle résulte le blocage mécanique de l'organe de blocage (452) par l'organe de verrouillage.

11. Dispositif de distribution selon la revendication 10, **caractérisé en ce que** la force exercée par l'aimant permanent (446) dans la position de blocage de l'organe de blocage (452) sur l'organe de blocage (452), l'organe auxiliaire de blocage, l'organe de verrouillage et/ou l'organe auxiliaire de verrouillage est plus élevée que la force agissant en direction opposée par les moyens de ressort du dispositif de distribution dans la position de blocage de l'organe de blocage sur l'organe de blocage, l'organe auxiliaire de blocage, l'organe de verrouillage ou l'organe auxiliaire de verrouillage.

12. Dispositif de distribution selon l'une quelconque des revendications 7 à 11, **caractérisé en ce qu'**il est prévu, pour produire le déclenchement, un actionneur à commande électrique, de préférence un électroaimant, par lequel l'organe de blocage ou un organe auxiliaire de blocage relié opérativement à l'organe de blocage peut être soumis à une force, de telle manière qu'il en résulte que l'organe de blocage est déplacé en direction de sa position de libération, ou un organe de verrouillage ou un organe auxiliaire de verrouillage relié opérativement à l'organe de verrouillage est soumis à une force dans une direction qui conduit à une libération du blocage mécanique de l'organe de blocage (452) par l'organe de verrouillage et dès lors à un déplacement de l'organe de blocage (452) en direction de sa position de libération.

13. Dispositif de distribution selon l'une quelconque des revendications précédentes, **caractérisé en ce que** l'organe de blocage (452) est réalisé en mode rotatif autour d'un axe de rotation (1) orienté dans la direction d'actionnement (1a) pour le transfert de la position de blocage à la position de libération et présente au moins trois segments de blocage (462) répartis sur sa périphérie, qui sont disposés, dans la position de blocage de l'organe de blocage (452), de telle manière qu'un mouvement de course de l'organe d'actionnement (488) soit empêché.

14. Dispositif de distribution selon la revendication 13, **caractérisé en ce que** l'organe de blocage (452) entoure en périphérie l'organe d'actionnement (488) et l'axe de rotation (1) de l'organe de blocage (452) est orienté parallèlement à la direction d'actionnement (1a) et est de préférence coaxial à un axe central (1) du dispositif de distribution.
